# EUROPÄISCHE PATENTSCHRIFT

(11) **EP 0 527 703 B1**
(45) Veröffentlichungstag und Bekanntmachung des Hinweises auf die Patenterteilung: **28.06.1995**
(21) Anmeldenummer: 92810583.2
(22) Anmeldetag: 31.07.1992
(51) Int. Cl.: A61B 5/00

(54) **Einrichtung zur Messung mindestens einer Gassättigung, insbesondere der Sauerstoffsättigung von Blut**
Device for measuring at least one gaseous concentration level in particular the oxygen concentration level in blood
Dispositif permettant de déterminer la concentration d'au moins un composé gazeux en particulier la teneur en oxygène du sang

(30) Priorität: 12.08.1991 CH 2372/91
(43) Veröffentlichungstag der Anmeldung: 17.02.1993
(73) Patentinhaber: AVL Medical Instruments AG, 8207 Schaffhausen (CH)
(72) Erfinder: Hatschek, Rudolf Alexander, Dr., CH-1700 Fribourg 5 (CH)
(74) Vertreter: Krause, Walter, Dr. Dipl.-Ing.

(56) Entgegenhaltungen:
- WO-A-91/11136
- GB-A- 2 228 314
- US-A- 4 890 619
- US-A- 5 066 859
- PROC. OF THE 7TH. ANNUAL CONF. OF THE IEEE/ENGINEERING IN MEDICINE AND BIOLOGYSOCIETY Bd. 1, 27. September 1985, CHICAGO (US) Seiten 144 - 147 T. M. DONAHOEET AL. 'A NEW NONINVASIVE BACKSCATTERING OXIMETER'

## Beschreibung

Die Erfindung betrifft eine Einrichtung zur Messung mindestens einer Gassättigung, insbesondere der Sauerstoffsättigung, von in einem lebenden Körper vorhandenem Blut. Die Einrichtung kann auch ausgebildet sein, um zusätzlich zur Sauerstoffsättigung oder möglicherweise anstelle von dieser eine andere Gassättigung von Blut - etwa dessen Kohlenmonoxydsättigung - zu messen. Die Einrichtung soll dabei eine nicht-invasive Messung ermöglichen. Der Begriff "nicht-invasiv" besagt hierbei, dass die Messung ohne irgend ein in ein Blutgefäss eingeführtes Instrument und also ausschliesslich mit Sensormitteln durchgeführt wird, die sich vollständig ausserhalb des lebenden, menschlichen oder eventuell tierischen Körpers befinden, an dem die Messung durchgeführt wird. Bei den vorgesehenen Verwendungen der Einrichtung zum Messen einer Gassättigung will man meistens diejenige des arteriellen Blutes messen.

Die vor allem in den roten Blutkörperchen des Blutes vorhandenen Hämoglobine können verschiedene Gase - wie Sauerstoff und Kohlenmonoxid - binden, transportieren und wieder abgeben. Das Blut kann insbesondere Oxyhämoglobin, d.h. Hämoglobin mit gebundenem Sauerstoff, Desoxyhämoglobin, d.h. Hämoglobin ohne Sauerstoff, und Kohlenmonoxidhämoglobin enthalten. Unter einer Gassättigung wird ein Wert verstanden, der ein Mass für die Konzentration oder den Anteil des Hämoglobins gibt, welches das betreffende Gas enthält. Zum Beispiel kann die insbesondere zu messende Sauerstoffsättigung ein Mass für das Verhältnis zwischen der Konzentration des Oxyhämoglobins, und der Konzentration der Summe von diesem sowie dem Desoxyhämoglobin oder der gesamten Hämoglobinkonzentration geben. Als Mass der Sauerstoffsättigung wird häufig der Sättigungsgrad angegeben, der gleich dem in Prozenten dargestellten Wert des genannten Verhältnisses ist.

Für die Messung der Sauerstoffsättigung oder eines andern Gasgehaltes von Blut ist von Bedeutung, dass in einen Körper und insbesondere in ein Blutgefäss eingestrahltes Licht gestreut wird. Das Blutplasma und die in diesem enthaltenen, roten Blutkörperchen sowie sonstigen Blutzellen haben verschiedene Brechungsindizes, so dass ein Lichtstrahl beim Eintritt in eine beispielsweise durch ein rotes Blutkörperchen gebildete Blutzelle und beim Austritt aus einer solchen normalerweise durch Refraktion umgelenkt wird. Ferner kann das Licht auch durch Reflexion und Beugung umgelenkt werden. Die Gesamtheit aller Umlenkungsprozesse wird als Streuung bezeichnet. Ein in ein Blutgefäss eingestrahlter Lichtstrahl wird meistens mehrere Male im Blut gestreut, bis er wieder aus dem Blutgefäss austritt. Wenn das Licht ein rotes Blutkörperchen durchdringt, wird ein Teil des Lichtes absorbiert.

Beim Messen der Sauerstoffsättigung unter Verwendung von Licht wird von der Tatsache Gebrauch gemacht, dass das gebundenen Sauerstoff enthaltende Hämoglobin, d.h. das Oxyhämoglobin, und das sauerstofflose Hämoglobin, d.h. das Desoxyhämoglobin, verschiedene Farben und dementsprechend verschiedene Licht-Absorptionsspektren haben. Wenn die Absorptionskoeffizienten der beiden Hämoglobinarten in Abhängigkeit der Lichtwellenlänge im gleichen Diagramm durch Kurven dargestellt werden, kreuzen die beiden Kurven einander in sogenannten isobestischen Punkten, zum Beispiel bei einer im folgenden als isobestische Wellenlänge bezeichneten, ungefähr 805 nm betragenden Wellenlänge.

Einrichtungen zur nicht-invasiven Messung von einer Gassättigung von Blut, insbesondere von dessen Sauerstoffsättigung, sind zum Beispiel aus der Publikation "A new noninvasive backscattering oximeter", T.M. Donahoe und R.L. Longini, Proceedings of the Seventh Annual Conference of the IEEE/Engineering in Medicine and Biology Society, 1985, Chicago, USA, Band 1, Seiten 144 bis 147, aus der US-A-4 890 619 und aus der GB-A-2 228 314 bekannt. Diese Einrichtungen besitzen Sensormittel, Lichtstrahlungsmittel, um bei mindestens einem Lichtstrahlungsbereich der Sensormittel Licht in einen zu untersuchenden Körper einzustrahlen, und Lichtempfangsmittel. um bei mindestens einem Lichtempfangsbereich der Sensormittel im Körper gestreutes Licht zu empfangen und mit mindestens einem Fotohalbleiter dessen Intensität zu messen. Die bekannten Einrichtungen besitzen ferner elektronische Schaltungsmittel, um aus den von den Fotohalbleitern ermittelten Lichtintensitäten die Sauerstoffsättigung und eventuell andere Grössen zu ermitteln.

Die aus der zitierten Publikation von Donahoe et al. bekannte Einrichtung weist zwei identische Sensoren auf. Jeder von diesen besitzt zwei Leuchtdioden, die Lichtquellen zur Erzeugung von Licht mit zwei verschiedenen Wellenlängen bilden und auf der beim Messen am zu untersuchenden Körper anliegenden Seite des Sensors zusammen einen Lichtstrahlungsbereich definieren, durch welchen das erzeugte Licht in den Körper gestrahlt werden kann. Jeder Sensor besitzt ferner Lichtempfangsmittel mit zwei je aus einem Fototransistor bestehenden Lichtempfängern. Die beiden Fototransistoren stehen in einer Draufsicht auf die zum Anliegen am Körper bestimmte Seite des Sensors in verschiedenen Abständen von einer durch die beiden Leuchtdioden verlaufenden Geraden und definieren je einen Lichtempfangsbereich, durch den im Körper zurückgestreutes Licht zum betreffenden Fototransistor gelangen kann. Bei der Durchführung einer Messung wird der eine, zur Messung der Sauerstoffsättigung des arteriellen Blutes vorgesehene Sensor mit einer in diesem vorhandenen Heizwicklung erwärmt, während der andere Sensor nicht erwärmt wird und die Sauerstoffsättigung des Gewebes messen soll.

Die aus der schon zitierten US-A-4 890 619 bekannten Einrichtungen besitzen auch zwei identisch ausgebildete Sensoren, von denen jeder einen durch eine Fotodiode gebildeten Lichtempfänger und vier oder sechs auf einer diesen umschliessenden Kreislinie angeordnete Leuchtdioden aufweist. Die Leuchtdioden jedes Sensors bilden Lichtquellen zur Erzeugung von Licht mit zwei verschiedenen Wellenlängen und definieren zusammen einen kreisringförmigen Lichtstrahlungsbereich, bei welchem Licht aus dem Sensor herausgestrahlt werden kann. Der Abstand des von der Fotodiode des betreffenden Sensors definierten Lichtempfangsbereichs vom ringförmigen Lichtstrahlungsbereich ist etwa gleich dem mittleren Radius des letzteren. Die Sensoren weisen zum Teil noch Ultraschallquellen zum Erwärmen des Gewebes durch Ultraschall auf. Die aus der US-A-4 890 619 bekannten Einrichtungen ermöglichen, die Sauerstoffsättigung abhängig von der Anordnung der beiden Sensoren mit den Körper durchdringendem, von einem Sensor zum andern Sensor gelangendem Transmissionslicht oder mit Rückstreulicht zu messen, das vom Körper in den gleichen Sensor zurückgestreut wird, von dem es ausgestrahlt wurde.

In der bereits zitierten GB-A-2 228 314 ist eine Einrichtung offenbart, die unter anderem zur Messung der Sauerstoffsättigung des im Gehirn vorhandenen Blutes ausgebildet ist. Die Lichtstrahlungsmittel der Einrichtung besitzen eine Anzahl Laserdioden, die für die Messung der Sauerstoffsättigung Licht mit verschiedenen Wellenlängen erzeugen können. Jede Laserdiode ist mit dem Eintrittsende eines Lichtleiters verbunden, dessen Austrittsende sich bei der Oberfläche des Kopfs eines zu untersuchenden Patienten befindet. Die Lichtempfangsmittel der Einrichtung besitzen eine Anzahl Lichtleiter mit sich bei der Oberfläche des Kopfs des Patienten befindenden Eintrittsenden. Bei der hauptsächlich beschriebenen Einrichtung führen die Lichtleiter der Lichtempfangsmittel vom Kopf alle zu ein und demselben Fotodetektor. Es ist noch erwähnt, dass für jeden Lichtleiter der Lichtempfangsmittel ein separater Fotodetektor vorgesehen werden könnte und dass der zu den elektronischen Schaltungsmittel der Einrichtung gehörenden Rechner die beim Messen von den verschiedenen Fotodetektoren ermittelten Lichtintensitäten dann addiert. Die Austrittsenden der zu den Lichtstrahlungsmitteln gehörenden Lichtleiter und die Eintrittsenden der zu den Lichtempfangsmitteln gehörenden Lichtleiter sind mehr oder weniger gleichmässig über die obere Hälfte des Kopfes der zu untersuchenden Person verteilt. Die Einrichtung besitzt daher eine Vielzahl von Lichtstrahlungsbereichen und Lichtempfangsbereichen, wobei die Lichtstrahlungsbereiche und die Lichtempfangsbereiche einer vertikal von oben her gesehenen Draufsicht entlang dem Umfang des Kopfes abwechselnd aufeinander folgen. Im übrigen sind zwischen den einander am nächsten benachbarten Lichtstrahlungsbereichen wie auch zwischen den einander am nächsten benachbarten Lichtempfangsbereichen relativ grosse Zwischenräume vorhanden. Beim Betrieb der Einrichtungen wird sequentiell Licht bei den verschiedenen Lichtstrahlungsbereichen durch den Schädel hindurch in das Gehirn gestrahlt. In diesen gestreutes und wieder durch den Schädel hindurch aus dem Kopf herausgestrahltes Licht kann dann bei den verschiedenen Lichtempfangsbereichen aufgefangen werden. Wenn zum Bespiel bei einer sich beim "Äquator" des Kopfes befindenden Lichtstrahlungsstelle Licht in den Kopf gestrahlt wird, kann bei jeder Lichtempfangsstelle gestreutes Licht aufgefangen werden, das abhängig von der Lage der betreffenden Lichtempfangsstelle zurückgestreut, auf die Seite gestreut oder vorwärtsgestreut wurde.

Beim Messen mit allen bekannten und vorgängig beschriebenen Einrichtungen gelangt nicht nur von den roten Blutkörperchen gestreutes Licht, sondern auch Licht dem bzw. jedem Lichtempfänger, das von abgestorbenen Hautzellen, Geweben, Haarfollikeln, Schweissdrüsen, Nerven usw. gestreut wurde. Dieses letztgenannte, nicht von Hämoglobinen gestreute Licht gibt keine Informationen über die zu messende Sauerstoffsättigung und beeinträchtigt die Messgenauigkeit. Für eine genaue Bestimmung der arteriellen Sauerstoffsättigung sollte daher ein möglichst grosser Teil des in den oder die Lichtempfänger gelangenden und für die Bestimmung der Sauerstoffsättigung verwerteten Lichtes von den roten Blutkörperchen gestreut sein. Wie noch eingehender erörtert wird, hängt die Intensität des von roten Blutkörperchen gestreuten und durch einen Lichtempfangsbereich in einen Lichtempfänger gelangenden Lichtes beim Messen mit Rückstreulicht vom entlang der Körperoberfläche gemessenen Abstand des Lichtempfangsbereichs vom Lichtstrahlungsbereich ab, bei dem das Licht in den Körper gestrahlt wurde. Wie ebenfalls noch näher erläutert wird, hängt der günstigste Wert dieses Abstandes von individuellen anatomischen Eigenschaften einer untersuchten Person, von der zum Messen gewählten Körperstelle und auch vom momentanen, zeitlichen Veränderungen unterworfenen, physiologischen Zustand einer untersuchten Person ab. Die aus den verschiedenen zitierten Publikationen bekannten Einrichtungen haben daher beim Messen mit Rückstreulicht die Nachteile, dass die erzielbare Messgenauigkeit von Messung zu Messung ändern kann und dass manchmal beträchtliche Messfehler auftreten.

Wenn die aus der US-A-4 890 619 bekannten Einrichtungen zum Messen mit Transmissionslicht verwendet werden, haben sie nebst mehr oder weniger ähnlichen Nachteilen wie beim Messen mit zurückgestreutem Licht vor allem den Nachteil, dass die Messungen nur an dünnen Körperteilen, wie etwa einem Ohrläppchen oder Finger durchgeführt werden können. Zudem sind Messungen mit Transmissionslicht relativ schlecht für langdauernde, kontinuierliche Überwachungen geeignet, weil zum Beispiel Bewegungen der untersuchten Person häufig Messfehler verursachen. Ähnliches gilt auch für die Messungen mit den Einrichtungen gemäss der GB-A-2 228 314. Da das Licht beim Betrieb dieser Einrichtungen zweimal Knochenteile des Schädels durchdringen muss, sind die messbaren Lichtintensitäten zudem sehr gering, was die Messgenauigkeit zusätzlich beeinträchtigt.

Wenn bei den bekannten Einrichtungen die Strahlungsintensitäten der verschiedenen Leucht- oder Laserdioden oder die Empfindlichkeit der Photohalbleiter im Lauf der Zeit ändern, kann dies die Messgenauigkeit ebenfalls beeinträchtigen. Dabei können insbesondere dann Messungenauigkeiten auftreten, wenn die Strahlungsintensität bei den verschiedenen Wellenlängen unterschiedlich ändert oder wenn die spektrale Empfindlichkeit eines Photohalbleiters ändert.

Wir erwähnt, sind die Sensoren der Einrichtung gemäss der zitierten Publikation von Donahoe et al. mit einer Heizwicklung ausgerüstet. Diese ermöglich, die Haut durch Wärmeleitung zu erwärmen und dadurch die Durchblutung zu vergrössern. Wenn der Arterien und Arteriolen enthaltende Bereich der Haut - d.h. die Dermis - durch Wärmeleitung von der Hautoberfläche her erwärmt wird, entsteht in der Haut ein Temperaturgefälle von aussen nach innen. Wenn die Erwärmung ausschliesslich durch Wärmeleitung erfolgt, ist es daher schwierig und praktisch unmöglich, den die Arterien und Arteriolen enthaltenden Hautbereich auf eine zum Messen der arteriellen Sauerstoffsättigung optimale Temperatur zu erwärmen, ohne in gewissen Hautbereichen schädigende Überhitzungen zu verursachen.

Bei denjenigen aus der US-A-4 890 619 bekannten Sensoren, die mit Ultraschallquellen ausgerüstet sind, ist zwischen den im Innern eines Gehäuses des Sensors angeordneten Ultraschallquellen und der Hautoberfläche des untersuchten Patienten ein ziemlich grosser Hohlraum vorhanden. Dieser muss zum Messen entweder mit einer gallertartigen und mehr oder weniger gut fliessfähigen Füllmasse aus Polyäthylenglykol oder mit Wasser gefüllt werden, was den Messvorgang komplizierter macht und im Fall der Verwendung von Wasser eine zum Einbringen von diesem dienende Pumpe erfordert. Da der in die Haut eingestrahlte Ultraschall in dieser nicht überall gleichmässig absorbiert wird, kann eine ausschliesslich durch das Einstrahlen von Ultraschall in die Haut erfolgende Erwärmung zudem wie eine ausschliesslich durch Wärmeleitung erfolgende Erwärmung zu einer zwar andern, aber ebenfalls ungleichmässigen Temperaturverteilung führen.

Die Sauerstoffversorgung von Geweben und/oder Organen eines Lebewesens hängt nicht nur von der Sauerstoffsättigung des Blutes, sondern auch von der Intensität der Blut-Perfusion, d.h. der Durchblutung ab. Beim Messen der Sauerstoffsättigung ist es daher wünschenswert, gleichzeitig die Stärke der Blut-Perfusion zu messen. Mit den bekannten, zur Messung der Sauerstoffsättigung dienenden Einrichtungen kann jedoch die Blut-Perfusion nicht oder höchstens mit geringer Genauigkeit gemessen werden.

Ähnliche Probleme wie beim Messen der Sauerstoffsättigung können sich auch beim Messen einer andern Gassättigung, zum Beispiel der Kohlenmonoxydsättigung von Blut stellen.

Der Erfindung liegt daher die Aufgabe zugrunde, eine Einrichtung zur Messung mindestens einer Gassättigung von Blut - insbesondere von dessen Sauerstoffsättigung - zu schaffen, die Nachteile der bekannten Einrichtungen behebt. Dabei wird insbesondere angestrebt, beim Messen unabhängig von individuellen anatomischen Eigenschaften des Körpers eines untersuchten Lebewesens, unabhängig von der für die Messung gewählten Stelle des Körpers und unabhängig von physiologisch bedingten Zustandsänderungen eine möglichst grosse Messgenauigkeit zu erzielen.

Die gestellte Aufgabe wird gemäss der Erfindung durch eine Einrichtung mit den Merkmalen des Anspruchs 1 gelöst. Vorteilhafte Ausgestaltungen der Einrichtung gehen aus den abhängigen Ansprüchen hervor.

Die Streuung des für die Bestimmung einer Gassättigung - insbesondere der Sauerstoffsättigung - erwünschten, zeitlich periodisch im Takt der Pulsfrequenz ändernden Streulichts erfolgt vorherrschend in den in der mittleren und/oder tieferen Dermis vorhandenen Arterien. Dagegen ist die Intensität von Streulicht, das ausschliesslich bei Streuvorgängen in den über den Arterien liegenden Hautschichten gestreut wurde, überwiegend zeitlich konstant und/oder zeitlich nur mehr oder weniger stochastisch ändernd. Das Streulicht mit einer zeitlich konstanten und/oder stochastisch ändernden Intensität kann als Wirkung einer Hintergrund-Streuung und/oder eines physiologischen Rauschens aufgefasst und als Hintergrund- und/oder Rausch-Rückstreulicht bezeichnet werden. Die Intensität, Zusammensetzung und Richtung des aus der Hautoberfläche austretenden Streulichts hängt von der Dicke, der Struktur sowie den Absorptionseigenschaften der verschiedenen Hautschichten, dem Hämatocrit-Wert und der Durchblutung ab. Der Hämatocrit-Wert bezeichnet das Verhältnis zwischen dem Volumen der roten Blutkörperchen und dem Volumen des Vollblutes. Zum Hämatocrit-Wert sei noch angemerkt, dass dieser üblicherweise in Prozenten angegeben wird, typischerweise ungefähr 42% beträgt, jedoch zwischen 20% bis 60% variieren kann.

Die in der Haut stattfindende Streuung wurde für ein scharf gebündeltes, bei einer punktartigen Lichtstrahlungsstelle in die Haut gestrahltes Lichtstrahlenbündel mit Modellrechnungen und experimentell untersucht. Für die Modellrechnungen wurde die Haut in vier Schichten mit verschiedenen Streu- und Absorptionseigenschaften unterteilt. Dabei wurde je eine Schicht der Hornhaut, der Keimschicht, der äussern Schicht der Dermis bzw. der Arterien aufweisenden, mittleren und/oder tieferen Schicht der Dermis zugeordnet. Bei den Modellrechnungen wurden verschiedene Modelle verwendet. Bei einem relativ einfachen Modell wurde jede Schicht durch eine ebene Fläche und eine mathematischen Funktion dargestellt, die jedem Punkt der Fläche eine Rückstrahlintensität zuordnet. Bei einem der Wirklichkeit näherkommenden, komplizierteren Modell wurden die vier Schichten als Kontinua aufgefasst und die Streuung durch die Diffusion von Photonen dargestellt und berechnet. Beide Modellrechnungen und die experimentellen Untersuchungen ergaben, dass die Intensität des aus der Haut heraus zurückgestreuten, konstanten Streulichts und des zeitlich stochastisch ändernden Streulichts beim der Lichtstrahlungsbereich ein Maximum hat und mit wachsender, entlang der Hautoberfläche gemessener Entfernung des Lichtempfangsbereichs vom Lichtstrahlungsbereich abnimmt. Die den Verlauf der Intensität in Abhängigkeit von der Entfernung darstellende Kurve hat dabei ungefähr die Form einer eine Gauss-Verteilung darstellenden Glockenkurve. Es wurde ferner gefunden, dass die Intensität des in der mittleren und/oder tieferen Schicht der Dermis gestreuten Lichtes und auch das Verhältnis zwischen dieser Intensität und der Intensität des gesamten Streulichts mit wachsender Entfernung des Lichtempfangsbereichs vom Lichtstrahlungsbereich zunimmt und danach wieder abnimmt. Die Intensität des in der mittleren und/oder tieferen Schicht der Dermis gestreuten Lichtes und das Verhältnis zwischen dieser Intensität und der Intensität des gesamten Streulichts haben also in einem gewissen Abstand vom Lichtstrahlungsbereich ein Maximum.

Die Untersuchungen ergaben des weitern, dass die Abstände dieser Maxima der Intensität des in der mittleren und/oder tieferen Schicht der Dermis gestreuten Lichtes von den Schichtdicken, den Tiefen der Arterien, den Streu- und Absorptionseigenschaften der verschiedenen Schichten und vom Hämatocrit-Wert abhängig sind. Da die Streuung und Absorption auch von der Durchblutung, d.h. vom Volumen des im untersuchten Körperabschnitt vorhandenen Blutes abhängig sind, kann die Lage der Maxima auch noch von der Durchblutung abhängig sein. Da die Dicken der verschiedenen Hautschichten, die Streu- und Absorptionseigenschaften sowie der Hämatocrit-Wert von Individuum zu Individuum verschieden sein können, von der zum Messen gewählten Körperstelle abhängig sind und zum Teil sogar zeitlich ändern können, sind auch die Abstände, bei denen die genannten Maxima auftreten, verschieden.

Gemäss der Erfindung besitzt die Einrichtung daher einen Sensor mit mehreren Lichtempfangsbereichen, bei denen die Lichtintensitäten separat gemessen werden können. Die Lichtempfangsbereiche haben in einer Draufsicht auf die zum Anliegen an einem lebenden Körper bestimmte Seite des Sensors und also beim Messen entlang der Körperoberfläche gemessen, an welcher der Sensor anliegt, verschiedene Abstände vom Lichtstrahlungsbereich des Sensors.

Die erfindungsgemässe Einrichtung weist ferner Schaltungsmittel, nämlich elektronische Schaltungsmittel auf, die beim Messen aufgrund eines vorgegebenen Auswahl-Kriteriums mindestens einen günstigen Lichtempfangsbereich als Verwertungsbereich auswählen. Die beispielsweise einen Mikroprozessor aufweisenden Schaltungsmittel können zum Beispiel ausgebildet sein, um die Pulsfrequenz zu ermitteln und den zeitlich periodisch im Takt der letzteren ändernden Teil des Streulichts für die Bestimmung der Gassättigung - beispielsweise der Sauerstoffsättigung - der Hämoglobine des arteriellen Blutes zu verwenden. Die Schaltungsmittel können dann zum Beispiel die Gassättigung aus den bei demjenigen Lichtempfangsbereich gemessenen Lichtintensitäten ermitteln, bei dem der Hub und damit auch die Amplitude der im Takt der Herzschläge ändernden Lichtintensität für eine der verwendeten Lichtwellenlängen am grössten ist oder bei dem das Verhältnis zwischen der genannten Amplitude und der Intensität des gesamten, die genannte Wellenlänge aufweisenden Streulichts am grössten ist.

Der Erfindungsgegenstand wird anschliessend anhand in der Zeichnung dargestellter Ausführungsbeispiele erläutert. In der Zeichnung zeigt
die Fig. 1 einen schematischen, nicht-massstäblichen Schnitt durch ein Stück der Haut eines lebenden Körpers und eine Einrichtung zur Messung der Sauerstoffsättigung mit einem im Schnitt gezeichneten, an der Haut anliegenden Sensor und mit einer mit diesem verbundenen, in Ansicht gezeichneten Messvorrichtung,
die Fig. 2 eine Draufsicht auf den in der Fig. 1 ersichtlichen Sensor,
die Fig. 3 eine axonometrische Darstellung eines Ausschnitts der einen im Sensor vorhandenen Lichtleitmittel in wesentlich grösserem Massstab als die in den Fig. 1 und 2 gezeichneten Sensoren,
die Fig. 4 eine Ansicht der sich in der Fig. 1 auf der untern Seite des im Sensor vorhandenen piezoelektrischen Elements befindenden Elektrode,
die Fig. 5 eine vereinfachte Ansicht der zum Zuwenden zum Sensor bestimmten Seite des Eich-Lichtverteilers der Einrichtung,
die Fig. 6 ein Blockschema der in der Fig. 1 ersichtlichen Einrichtung.
die Fig. 7 eine der Fig. 1 entsprechende Darstellung mit einer Variante der Einrichtung,
die Fig. 8 eine Draufsicht auf den Sensor der in der Fig. 7 gezeichneten Einrichtung und
die Fig. 9 eine Draufsicht auf eine integrierte Schaltung mit mehreren als Lichtempfängern dienenden Fotodioden.

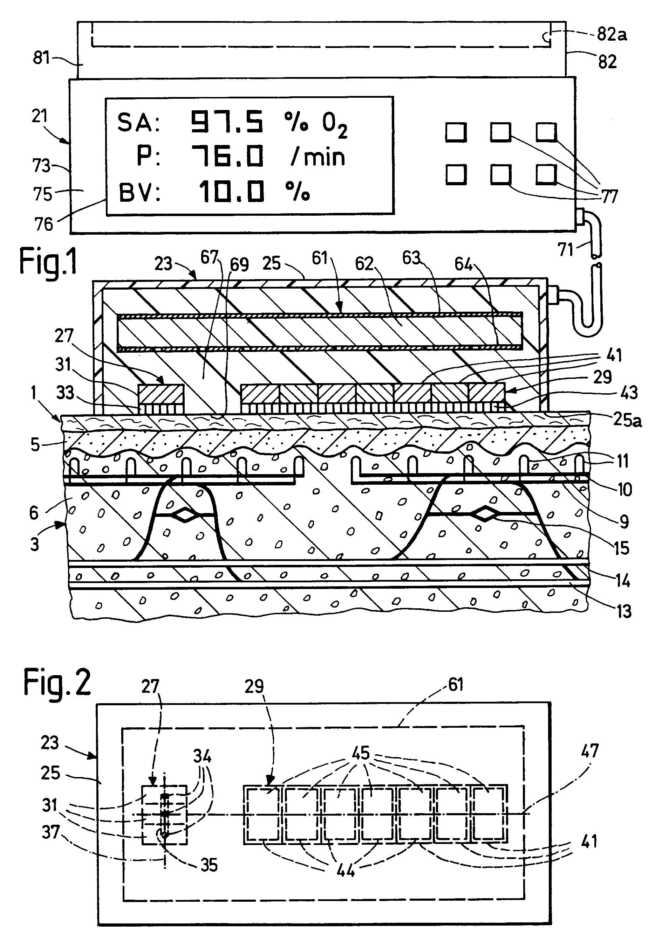

In der Fig. 1 ist ein Stück der zu einem Körper 1 eines Patienten gehörenden Haut 3 ersichtlich. Diese besteht aus der gefässlosen Epidermis 5 und der Blutgefässe enthaltenden Dermis 6. Die Epidermis 5 kann in mindestens zwei Schichten, nämlich die Hornhaut und die Keimschicht unterteilt werden, von denen die aus abgestorbenen Zellen bestehende Hornhaut die Oberfläche des Körpers 1 bildet. Die äussere oder obere Schicht der Dermis 6 enthält die kleinsten, die Mikrozirkulation ermöglichenden Blutgefässe, nämlich Arteriolen 9, Venolen 10 und Kapillarschleifen 11. Die Arteriolen 9 und Venolen 10 sind mit in der mittleren und/oder tieferen Schicht der Dermis 6 liegenden, grösseren Arterien 13 und Venen 14 verbunden. Ferner enthält die Dermis noch arteriovenöse Anastomosen 15, die steuerbare Verbindungen zwischen einer Arteriole und einer Venole bilden, zur Steuerung der Durchblutung dienen und in geöffnetem Zustand die mit der betreffenden Arteriole bzw. Venole verbundenen Kapillargefässe überbrücken können.

In der Fig. 1 ist auch eine als Ganzes mit 21 bezeichneten Einrichtung zur Messung mindestens einer Gassättigung von Blut, nämlich mindestens der Sauerstoffsättigung von Blut ersichtlich. Die Einrichtung 21 weist Sensormittel mit einem und nur einem Sensor 23 auf, der bei seiner Benutzung zum Anliegen der Oberfläche der Haut 3 gebracht wird. Der Sensor kann wahlweise an einer Vielzahl verschiedener Körperstellen angeordnet werden. Der Sensor 23 kann mit nicht gezeichneten Befestigungsmitteln - etwa mit mindestens einem Klebstreifen - lösbar an der Haut 3 befestigt werden.

Der auch separat in der Fig. 2 gezeichnete, im wesentlichen formfeste Sensor 23 weist ein Gehäuse 25 auf, das beispielsweise ungefähr die Form eines länglichen Quaders hat, aber statt Kanten abgerundete Übergänge aufweisen könnte. Das zum Beispiel aus Kunststoff bestehende Gehäuse 25 ist auf seiner beim Messen der Haut 3 zugewandten Seite offen und hat dort einen umlaufenden Gehäuserand 25a, der eine ebene Fläche definiert. Auf seinen übrigen Seiten ist das Gehäuse überall geschlossen.

Im Gehäuse sind Lichtstrahlungsmittel 27 und Lichtempfangsmittel 29 angeordnet. Die Lichtstrahlungsmittel 27 weisen mehrere - nämlich drei - Lichtquellen 31 auf. Diese sind durch Halbleiterdioden, d.h. durch Leuchtdioden zur Erzeugung von "gewöhnlichem" Licht oder mindestens zum Teil durch Laserdioden zur Erzeugung von räumlich sowie zeitlich kohärentem Licht gebildet und können zum Beispiel aus GaAlAs-Dioden bestehen. Jede der drei Halbleiterdioden hat eine beim Messen der Haut 3 zugewandte Leuchtstelle und erzeugt beim Betrieb ein Lichtstrahlenbündel, dessen Öffnungs- oder Kegelwinkel zum Beispiel höchstens etwa 10° und dessen Durchmesser bei der Leuchtstelle der Diode zum Beispiel höchstens oder ungefähr 0,2 mm beträgt. Jede der drei Lichtquellen 31 bzw. Dioden kann impulsweise mindestens annähernd monochromatisches und beispielsweise kohärentes Licht erzeugen. Die drei Lichtquellen 31 sind jedoch zur Erzeugung von drei verschiedenen Lichtarten ausgebildet, von denen jede eine andere Wellenlänge hat. Die drei Lichtquellen 31 werden im folgenden als erste, zweite und dritte Lichtquellen bezeichnet, wobei diese Numerierung zum Beispiel mit der in der Fig. 2 von oben nach unten gezählten Reihenfolge übereinstimmen kann, dies aber nicht muss. Die erste Lichtquelle kann Licht mit einer in der Nähe der isobestischen Wellenlänge liegenden, zum Beispiel etwa 800 bis 830 nm betragenden Wellenlänge erzeugen. Die beiden andern Lichtquellen können dann Lichtarten erzeugen, deren Wellenlängen mindestens 100 nm verschieden von der isobestischen Wellenlänge, nämlich kleiner als diese sind. Die zweite Lichtquelle kann vorzugsweise Licht erzeugen, dessen Wellenlänge im Bereich von etwa 620 nm bis 750 nm liegt, in welchem die Absorptionskoeffizienten von Oxyhämoglobin und Desoxyhämoglobin stark verschieden voneinander sind. Die zweite Lichtquelle kann zum Beispiel Licht mit einer Wellenlänge von ungefähr 660 nm erzeugen. Die dritte Lichtquelle kann dann Licht erzeugen, dessen Wellenlänge im Bereich von etwa 500 nm bis 600 nm liegt, in dem sowohl Oxyhämoglobin als auch Desoxyhämoglobin und übrigens auch Hämoglobin mit gebundenem Kohlenmonoxyd eine relativ grosse Absorption ergibt. Die Wellenlänge des von der dritten Lichtquelle erzeugten Lichtes kann beispielsweise ungefähr 575 nm betragen.

Die drei Lichtquellen 31 bzw. Halbleiterdioden bestehen je aus einem separaten Bauelement und sind in einer geraden Reihe möglichst nahe nebeneinander angeordnet, so dass sie einander paarweise mindestens annähernd berühren. Im übrigen haben die Lichtquellen 31 zum Beispiel eine im allgemeinen viereckige, nämlich rechteckige Umrissform, wobei die längeren Rechteckseiten rechtwinklig zu einer zur Längsrichtung des Gehäuses 25 rechtwinkligen Geraden 37 verlaufen. Die Lichtstrahlungsmittel 27 weisen noch Lichtleitmittel 33 auf, die aus einem für alle Lichtquellen 31 gemeinsamen, noch näher beschriebenen plättchenförmigen Körper bestehen. Die Lichtleitmittel 33 haben eine den Lichtquellen 31 zugewandte sowie an diesen anliegende, ebene Lichteintrittsfläche und eine ebene Lichtaustrittsfläche, die mindestens ungefähr und nämlich genau in der vom Rand 25a des Gehäuses 25 aufgespannten Ebene liegt. Die drei Lichtquellen 31 definieren in einer Draufsicht auf die genannte Lichtaustrittsfläche je eine Lichtstrahlungsstelle 34, bei welcher das beim Betrieb von der betreffenden Lichtquelle erzeugte Licht aus dem dem Sensor heraus gestrahlt wird und die zum Beispiel aus einem kleinen kreiförmigen, fast punktartigen Feld besteht. Die drei Lichtstrahlungsstellen 34 werden von einer Hüll-Linie umhüllt, die einen ungefähr rechteckförmigen Lichtstrahlungsbereich 35 begrenzt. Die Mittelpunkte der in der genannten Draufsicht von den drei Halbleiterdioden 32 definierten Lichtstrahlungsstellen 34 liegen auf der Geraden 37. Der Mittelpunkt der mittleren Lichtstrahlungsstelle 34 bildet auch den Mittelpunkt des ganzen Lichtstrahlungsbereichts 35. Die entlang der Geraden 37 gemessenen Abmessungen der Lichtquellen 31 und die Abstände der Mittelpunkte der einander benachbarten Lichtstrahlungsstellen 34 betragen vorzugsweise höchstens 2 mm und beispielsweise ungefähr 1 mm bis 1,5 mm. Die entlang der Geraden 37 gemessene Ausdehnung des Lichtstrahlungsbereichs 35 beträgt dementsprechend höchstens 6 mm und vorzugsweise höchstens als 5 mm. Die rechtwinklig zur Geraden 37 gemessene Ausdehnung des Lichtstrahlungsbereichs 35 beträgt höchstens 2 mm vorzugsweise höchstens 1 mm und beispielsweise weniger als 0,5 mm.

Die Lichtempfangsmittel 29 besitzen mehrere, nämlich mindestens drei, vorzugsweise mindestens fünf und beispielsweise bis etwa zwanzig in einer geraden Reihe angeordnete Lichtempfänger 41, wobei für das Ausführungsbeispiel sieben Lichtempfänger gezeichnet sind. Diese bestehen aus separaten, identisch ausgebildeten Fotohalbleitern, nämlich Silicium-Fotodioden. Die Lichtempfangsmittel 29 sind mit Lichtleitmitteln 43 ausgerüstet, die aus einem für alle Lichtempfänger 43 gemeinsamen, plättchenförmigen Körper bestehen, der für jeden Lichtempfänger einen diesem zugeordneten Mehrfach-Lichtleiter und nicht-abbildenden Kollimator bildet. Die Lichtleitmittel 43 haben eine ebene Lichteintrittsfläche, die mindestens ungefähr und nämlich genau in der vom Rand 25a des Gehäuses 25 aufgespannten Ebene liegt, und eine ebene Lichtaustrittsfläche, die den Lichteintrittsseiten der Lichtempfänger 41 zugewandt ist und an diesen anliegt.

Die Lichtempfänger 41 sind entlang der von ihnen gebildeten Reihe mindestens annähernd lückenlos nebeneinander angeordnet. Jeder Lichtempfänger 41 definiert in einer Draufsicht in einer auf die beim Messen am Körper anliegenden Seite des Sensors und also in der Lichteintrittsfläche der Lichtleitmittel 43 ein Lichteinlassfeld 44, bei welchem Licht durch die Lichtleitmittel 43 hindurch in den betreffenden Lichtempfänger gelangen kann, so dass dieser Lichtempfänger dann die Intensität dieses Lichtes messen kann. Die Lichteinlassfelder 44 bilden entsprechend der Anordnung der Lichtempfänger 41 eine gerade Reihe und haben auf einer geraden Mittellinie 47 liegende Mittelpunkte. Die Mittellinie 47 verläuft parallel zur Längsrichtung des Gehäuses 25 und rechtwinklig zur Geraden 37 durch den Mittelpunkt der mittleren Lichtstrahlungsstelle 34 und des ganzen Lichtstrahlungsbereichs 35. Die Lichteinlassfelder 44 haben zum Beispiel eine im allgemeinen viereckige und nämlich rechteckige Umrissform, wobei die längeren Rechteckseiten parallel zur Geraden 37 sind. Im übrigen folgen die Lichteinlassfelder 44 der einander paarweise benachbarten Lichtempfänger mindestens annähernd lückenlos aufeinander. Abhängig von der Ausbildung der Gehäuse und der Lichteintrittsseiten der Lichtempfänger 41 können jedoch zwischen den einander paarweise benachbarten Lichteinlassfeldern 44 kleine Zwischenräume - d.h. Flächenabschnitte - vorhanden sein, bei denen das auf sie auftreffende Licht in keinen der Lichtempfänger gelangt. Die in der Längsrichtung der Reihe der Lichtempfänger gemessene Ausdehnung jedes Lichteinlassfeldes 44 soll jedoch mehr als die Hälfte und vorzugsweise mindestens zwei Drittel des Abstandes der Mittelpunkte von einander benachbarten Lichteinlassfeldern 44 betragen. Jedes von diesen dient als Lichtempfangsfeld 45, bei welchem beim Betrieb die Intensität des im Körper 1 zurückgestreuten und wieder in den Sensor gelangenden Lichtes separat gemessen werden kann.

Die verschiedenen Lichteinlassfelder 44 und die mit diesen identischen Lichtempfangsbereiche 45 haben verschiedene Abstände von den Lichtstrahlungsstellen 34, dem diese enthaltenden Lichtstrahlungsbereich 35 und der durch die Mittelpunkte der Lichtstrahlungsstellen 34 verlaufenden Geraden 37. Es ist anzumerken, dass diese Abstände rechtwinklig zu den Mittelachsen der bei den Lichtstrahlungsstellen 34 aus dem Sensor 23 herausgestrahlten Lichtstrahlenbündel und parallel zur Ebene gemessen werden, in der die Lichtstrahlungsstellen 34, der Lichtstrahlungsbereich 35 sowie die Lichtempfangsbereiche 45 liegen. Der Abstand des Mittelpunkts des sich am nächsten beim Lichtstrahlungsbereich 35 befindenden Lichteinlassfeldes 44 bzw. Lichtempfangsbereichs 45 von der Geraden 37 sowie vom Mittelpunkt des Lichtstrahlungsbereichs 35 beträgt vorzugsweise mindestens 4 mm, vorzugsweise höchstens 10 mm und zum Beispiel ungefähr 7 mm. Die parallel zur Geraden 47 gemessenen Abmessungen der Lichtempfänger 41 sowie Lichteinlassfelder 44 und die Abstände der Mittelpunkte einander benachbarter Lichteinlassfelder 44 bzw. Lichtempfangsbereiche 45 betragen vorzugsweise höchstens 3 mm und beispielsweise 1,5 mm bis 2,5 mm. Der Abstand des Mittelpunktes des am weitesten von den Lichtstrahlungsstellen 34 entfernten Lichteinlassfeldes 44 bzw. Lichtempfangsbereichs 45 von der Geraden 37 und vom Mittelpunkt des Lichtstrahlungsbereichs 35 beträgt vorzugsweise mindestens 15 mm und zum Beispiel 18 mm bis 30 mm. Die parallel zur Geraden 37 gemessene Abmessung - d.h. Länge - jedes Lichteinlassfeldes 44 ist vorzugsweise ungefähr oder mindestens gleich der in der gleichen Richtung gemessenen Abmessung des Lichtstrahlungsbereichs 35, d.h. der von den drei Lichtstrahlungsstellen 35 gebildeten Reihe.

Die zwei die Lichtleitmittel 33 und 43 bildenden, plättchenförmigen Körper haben in der in der Fig. 2 gezeichneten Draufsicht ungefähr oder genau die gleichen Umrissformen und Umrissabmessungen wie die Gruppe der Lichtquellen 31 bzw. die Gruppe der Lichtempfänger 41. Im übrigen sind die Lichtleitmittel 33 und 43 abgesehen von ihren unterschiedlichen Umrissformen und Umrissabmessungen gleich oder ähnlich ausgebildet.

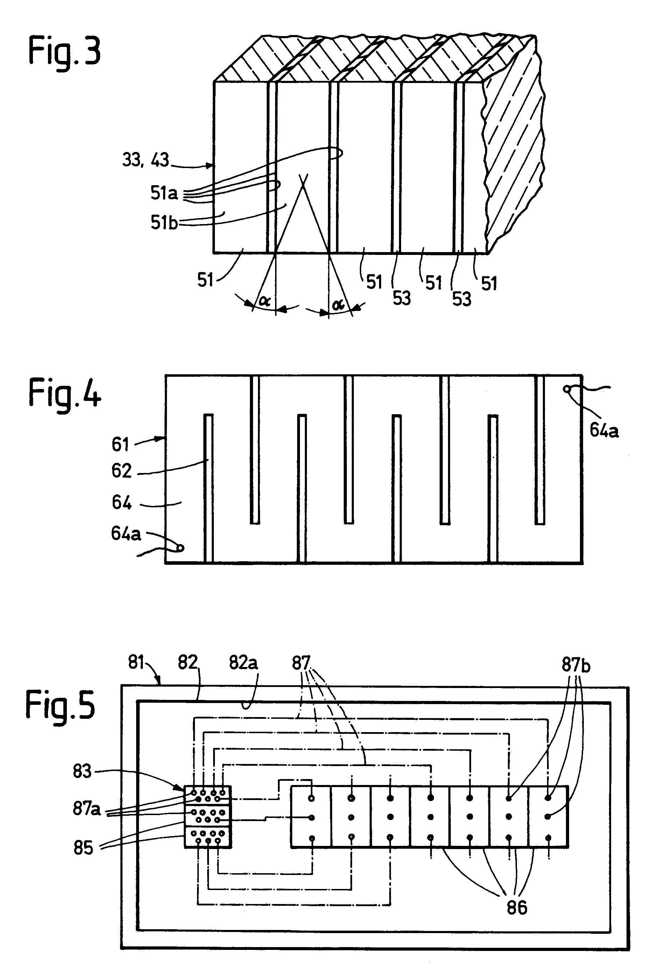

Die Ausbildung der Lichtleitmittel soll nun anhand der Fig. 3 beschrieben werden, die einen Ausschnitt aus den einen Lichtleitmitteln zeigt. Die Lichtleitmittel weisen eine Anzahl von ebenen, zueinander parallelen, zur Geraden 47 rechtwinkligen Scheiben 51 mit rechteckförmigem Umriss auf. Die bei den Breitseiten der Scheiben 51 vorhandenen Flächen von diesen sind mit 51a bezeichnet. Die entlang der Geraden 47 aufeinanderfolgenden Scheiben 51 sind durch zwischen den einander zugewandten Flächen 51a eines Scheibenpaares angeordnete Schichten 53 fest miteinander verbunden. Die Scheiben 51 haben bei allen Rändern, ebene, zu den Scheiben-Ebenen rechtwinklige Randflächen. Die Scheiben 51 sind glasklar sowie für die drei erzeugten Lichtarten möglichst gut lichtdurchlässig und bestehen zum Beispiel aus einem mineralischen Glas.

Die den Lichtquellen 31 zugewandten, ebenen Randflächen der Scheiben 51 der Lichtleitmittel 33 bilden zusammen die Lichteintrittfläche der letzteren. Die den Lichtquellen 31 abgewandten, ebenen Randflächen der Scheiben 51 der Lichtleitmittel 33 bilden zusammen deren Lichtaustrittsfläche, welche die Lichtstrahlungsstellen 34 und den Lichtstrahlungsbereich 35 enthalten. Hierzu ist anzumerken, dass die Durchmesser der von den Lichtquellen 31 erzeugten Lichtstrahlenbündel wesentlich kleiner sind als die parallel zur Geraden 47 gemessenen Abmessungen der Lichtleitmittel 33, so dass nur durch einen Teil von den zu diesen gehörenden Scheiben 51 oder sogar nur durch eine einzige Scheibe 51 der Lichtleitmittel 33 tatsächlich Licht hindurchgeleitet wird. Die den Lichtempfängern 41 abgewandten Randflächen der Scheiben der Lichtleitmittel 43 bilden zusammen deren Lichteintrittsfläche, die Lichteinlassfelder 44 und die Lichtempfangsbereiche 45. Die den Lichtempfängern 41 zugewandten Randflächen der Scheiben der Lichtleitmittel 43 bilden deren Lichtaustrittsfläche.

Die Schichten 53 sind aus einem Material gebildet, das mindestens zum Teil aus einem Klebstoff besteht, der mehr oder weniger gut lichtdurchlässig ist und einen kleineren Brechungsindex hat als das die Scheiben 51 bildende Glas. Der Klebstoff kann zum Beispiel durch einen Zwei-Komponenten-Klebstoff gebildet sein, dessen eine Komponente aus einem Epoxyharz besteht. Bei den Lichtleitmitteln 43 können eventuell diejenigen Schichten 53, welche die Grenze zwischen verschiedenen Lichteinlassfeldern 44 bilden, noch im eine lichtdurchlässige Matrix bildenden Epoxyharz dispergierte, stark lichtabsorbierende Partikel enthalten. Nötigenfalls können sogar alle Schichten 53 der Lichtleitmittel 33 und 43 solche Partikel enthalten. Diese erhöhen die Lichtabsorption der Schichten 53 und machen diese mehr oder weniger opak. Im übrigen können eventuell auch noch die sich an den einander abgewandten Enden der Scheiben-Reihen der Lichtleitmittel 33, 43 befindenden Flächen 51a der Scheiben 51 und die zu den Lichteintrittsflächen sowie den Lichtaustrittsflächen der Lichtleitmittel 33, 43 rechtwinkligen Randflächen 51b der Scheiben 51 mit einem Überzug versehen sein, der aus dem gleichen Material besteht, wie die Schichten 53. Es besteht jedoch auch die Möglichkeit die letztgenannten Randflächen der Scheiben mit einem Überzug zu versehen, der eine Verspiegelung ergibt und etwa aus einer aufgedampften Metallschicht besteht. Im übrigen sind die Lichtleitmittel 33, 43 elektrisch isolierend.

Die parallel zur Geraden 47 gemesse Dicke jeder Scheibe 51 ist wesentlich kleiner als die in der gleichen Richtung gemessenen Ausdehnungen jedes Lichteinlassfeldes 44 und jedes Lichtempfangsbereichs 45. Zudem ist die Dicke jeder Scheibe 51 kleiner als die rechtwinklig zu der Lichteintritts- und Lichtaustrittsfläche der Lichtleitmittel gemessene Höhe von diesen. Die Dicke der Scheiben 51 kann zum Beispiel etwa 0,15 mm bis 0,25 mm betragen. Die Höhe der Scheiben und der ganzen Lichtleitmittel kann etwa 1 mm bis 2 mm betragen. Die Dicke der Schichten 53 beträgt vorzugsweise höchstens 30% und zum Beispiel ungefähr oder höchsten 20% der Dicke der Scheiben 51.

Wenn durch die Lichteintrittsfläche der Lichtleitmittel 33 oder 43 in eine Scheibe 51 hineingelangtes Licht vom Innern der Scheibe her auf eine von deren Flächen 51a trifft, die eine optische Grenzfläche zwischen der betreffenden Scheibe 51 und einer Schicht 53 bildet, wird dieses Licht - abhängig vom Winkel zwischen der Grenzfläche und dem auf diese auftreffenden Licht - total oder nur zum Teil reflektiert. Eine Totalreflexion findet dabei dann statt, wenn der genannte Winkel gleich oder kleiner als der in der Fig. 3 mit alpha bezeichnete Winkel-Grenzwert ist. Dieser wird durch das Verhältnis des Brechungsindizes des die Scheiben bildenden Glases und des die Schichten 53 bildenden Materials bestimmt und kann zum Beispiel derart festgelegt werden, dass er höchstens 45° oder höchstens 30° oder höchstens 20° beträgt. Unter der Mittelebene der Lichtleitmittel 33 und/oder 43 wird im folgenden diejenige Ebene verstanden, die rechtwinklig zu den ebenen Lichteintritts- sowie Lichtaustrittsflächen ist und durch die Gerade 47 verläuft. Licht, welches parallel zur genannten Mittelebene in eine Scheibe 51 eingestrahlt wird und mit den Flächen 51a einen Winkel bildet, der höchstens gleich dem Winkel-Grenzwert alpha ist, kann die Scheibe ohne nennenswerte Abschwächung passieren. Dagegen wird Licht, das sich parallel zur genannten Mittelebene ausbreitet, aber mit den Flächen 51a einen mehr als alpha betragenden Winkel bildet, mehr oder weniger stark abgeschwächt. Die Scheiben 51 bilden also in Zusammenwirkung mit den Schichten 53 Lichtleiter und bewirken zudem - zumindest für parallel zur genannten Mittelebene gerichtetes Licht - eine Bündelung, d.h. eine nicht-abbildende Kollimation. Infolge dieser Kollimation gelangt nur solches Licht unabgeschwächt durch die Lichtleitmittel hindurch, dessen Richtung in einem Winkelbereich von 2 alpha liegt. Hingegen kann zur Geraden 47 rechtwinklige und also zur den Scheiben 51 parallele Ausbreitungsrichtungen aufweisendes Licht auch dann, wenn es mit einer senkrecht auf der Hautoberfläche stehenden Geraden einen grossen Winkel bildet, die Lichtleitmittel praktisch ohne Abschwächung passieren. Dabei haben die Lichtleitmittel für das Licht eine grosse Durchlassquerschnittsfläche. Es sei hierzu noch angemerkt, dass die kollimierende Wirkung bei den sich vor den Lichtquellen 31 befindenden Lichtleitmitteln 33 an sich unwesentlich ist, da die Lichtquellen 31 ohnehin Lichtstrahlenbündel mit fast parallelen Lichtstrahlen erzeugen.

Zur Herstellung der Lichtleitmittel 33 und 43 kann man zum Beispiel durch Verkleben von gläsernen Scheiben Blöcke bilden, welche im Grundriss die in der Fig. 2 gezeichneten Formen und Abmessungen der Lichtstrahlungsmittel 27 bzw. Lichtempfangsmittel 29 und eine Höhe haben, die ein Mehrfaches der in der Fig. 1 ersichtlichen Höhe der fertigen Lichtleitmittel beträgt. Von diesen Blöcken können dann plättchenförmige Körper mit den für die Lichtleitmittel vorgesehenen Höhen abgeschnitten werden.

Im Gehäuse 25 ist auf der dem Lichtstrahlungsbereich 35 und den Lichteinlassfeldern 44 sowie Lichtempfangsbereichen 45 abgewandten Seite der Lichtquellen 31 und Lichtempfänger 41 eine separat in der Fig. 4 gezeichnete Ultraschallquelle 61 angeordnet. Diese besteht aus einem die Form eines rechteckigen Plättchens aufweisenden, piezoelektrischen Element 62 und zwei aufeinander abgewandten Seiten von diesem angeordneten Elektroden 63, 64. Das piezoelektrische Element 62 ragt in der in der Fig. 2 gezeichneten, zu den Lichtempfangsbereichen rechtwinkligen Projektion allseitig über den Lichtstrahlungsbereich 35 und die Lichtempfangsbereiche 45 hinaus. Mindestens eine der Elektroden 63, 64, nämlich zum Beispiel die sich auf der den Lichtquellen 31 und Lichtempfängern 41 zugewandten Seite des piezoelektrischen Elements 62 befindende, auch in der Fig. 4 ersichtliche Elektrode 64 ist durch ein längliches, Wellen- und/oder mäanderförmiges Band gebildet, das den grössten Teil des Elements 62 bedeckt, aber gleichzeitig einen elektrischen, als Temperaturfühler verwendbaren Widerstand bildet und an beiden Enden mit einem Anschluss 64a versehen ist. Die Elektrode 63 ist gleich wie das Element 62 rechteckförmig und bedeckt dieses vollständig, könnte aber ebenfalls wellen- oder mäanderförmig sein sowie als Temperaturfühler dienen.

Ferner sind Ultraschallübertragungsmittel vorhanden, um beim Betrieb von der Ultraschallquelle 61 erzeugte Ultraschallwellen auf die Haut 3 des Körpers 1 zu übertragen. Diese Ultraschallübertragungsmittel weisen einen festen Ultraschallübertragungs-und Heizkörper 67 auf. Dieser ist durch ein in das Gehäuse 75 eingegossenes Giessharz gebildet, umschliesst die Ultraschallquelle 61 allseitig, bildet zusammen mit dieser und den verschiedenen Teilen der Lichtststrahlungsmittel sowie der Lichtemfangsmittel einen festen, kompakten, alle im Gehäuse vorhandenen Hohlräume vollständig sowie porenfrei ausfüllenden Block und erstreckt sich bis zu der vom Rand 25a des Gehäuses 25 definierten Ebene. Der Sensor 23 hat daher auf seiner zum Anliegen am lebenden Körper 1 bestimmten Seite eine vom Gehäuserand 25a, den Lichtleitmitteln 33, 43 sowie dem Ultraschallübertragungs- und Heizkörper 67 gebildete, ebene, glatte und kompakte - d.h. ausnehmungslose - Begrenzungs- und/oder Anliegefläche 69, welche den Lichtstrahlungsbereich 35 mit allen Lichtstrahlungsstellen 34 sowie alle Lichteinlassfelder 44 bzw. Lichtempfangsbereiche 45 enthält und vollständig umschliesst. Vor der Benutzung des Sensors 23 wird auf dessen zum Anliegen am Körper 1 bestimmte Begrenzungs- und/oder Anliegefläche 69 jeweils eine diese vollständig bedeckende, dünne zum Beispiel 0,001 mm bis 0,01 mm dicke, nicht gezeichnete, gallertartige, d.h. halbfeste/halbflüssige, etwa aus Polyäthylenglykol bestehende, für das zum Messen benutzte Licht durchlässige Ultraschallübertragungsschicht aufgebracht. Der erwähnte, vom Ultraschallübertragungs- und Heizkörper 67, den Lichtstrahlungsmitteln 27, den Lichtempfangsmitteln 29 sowie der Ultraschallquelle 61 gebildete Block ermöglicht zusammen mit der gallertartigen Ultraschallübertragungsschicht eine gute Anpassung des Ultraschallwellenwiderstandes der Ultraschallquelle 61 an denjenigen der Haut 3, so dass von der Ultraschallquelle 61 erzeugte Ultraschallwellen mit höchstens wenig Reflexionen auf die Haut 3 des Körpers 1 übertragen werden können. Der Ultraschallübertragungs- und Heizkörper 67 ist ferner derart ausgebildet, dass er einen Teil der von der Ultraschallquelle in Form von Ultraschallwellen erzeugte Energie absorbiert und in Wärme umwandelt. Dadurch kann der Ultraschallübertragungs- und Heizkörper 67 beim Betrieb auf eine zum Beispiel im Bereich von 37° C bis 43° C liegende, regelbare Temperatur erwärmt werden.

Die Lichtquellen 31, Lichtempfänger 41 und die Elektroden 63, 64 des Sensors 23 sind elektrisch über ein flexibles Kabel 71 mit einer in Abstand von der untersuchten Person angeordneten Messvorrichtung 73 verbunden. Diese besteht aus einem Gerät mit einem Gehäuse 75, an und/oder in dem eine Anzeigevorrichtung 76, und manuell betätigbare Schalt- und/oder Stellorgane 77 befestigt sind.

Die Einrichtung 21 weist einen in der Fig. 1 und separat in der Fig. 5 gezeichneten Eich-Lichtverteiler 81 auf. Der Eich-Lichtverteiler 81 besitzt einen Support 82, der zum Beispiel derart an der Decke des Gehäuses 75 befestigt ist, dass er von dessen Umgebung her zugänglich ist. Der Support 82 ist derart ausgebildet, dass der Sensor 23, wenn er nicht zum Messen benutzt wird, und insbesondere zum Eichen vorübergehend in einer definierten Position an und/oder im Support 82 angeordnet und beispielsweise in eine in diesem vorhandene Vertiefung 82a gesteckt und eventuell mit irgendwelchen nicht gezeichneten Befestigungsmitteln, etwa Rastmitteln, lösbar befestigt werden kann.

Der Eich-Lichtverteiler 81 ist mit Lichtverteilungsmitteln 83 versehen, um beim Eichen von den verschiedenen Lichtquellen 31 des Sensors 23 erzeugtes und bei den Lichtstrahlungsstellen 35 aus dem Sensor herausgestrahltes Licht in vorgegebener Weise auf die verschiedenen Lichteinlassfelder 44 bzw. Lichtempfangsbereiche 45 zu verteilen und bei diesen den Lichtempfängern 41 zuzuführen. Die Lichtverteilungsmittel 83 weisen zum Beispiel für jede zum Sensor 23 gehörende Lichtstrahlungsstelle 34 ein dieser beim Eichen gegenüberstehendes Lichtkopplungselement 85, für jeden zum Sensor 23 gehörenden Lichtempfangsbereich 45 ein diesem beim Eichen gegenüberstehendes Lichtkopplungselement 86 und eine Anzahl in der Fig. 5 nur zum Teil angedeuteter Lichtleiter 87 mit je einer Glasfaser auf. Jeder Lichtleiter 87 hat ein mit einem Lichtkopplungselement 85 verbundenes Ende 87a und ein mit einem Lichtkopplungselement 86 verbundenes Ende 87b. Die Lichtkopplungselemente 85, 86 können etwa je einen lichtdurchlässigen und beispielsweise glasklaren Körper aufweisen, der zum Beispiel aus einem Giessharz gebildet und an eine Gruppe von Lichtleiter-Enden 87a oder 87b angegossen ist. Jedes Lichtkopplungselement 85 ist durch mindestens einen Lichtleiter 87 und vorzugsweise durch mehrere solche mit jedem Lichtkopplungselement 86 verbunden. Dabei sind vorzugsweise alle Paare von Lichtkopplungselementen 85, 86 durch die gleiche Anzahl Lichtleiter miteinander verbunden.

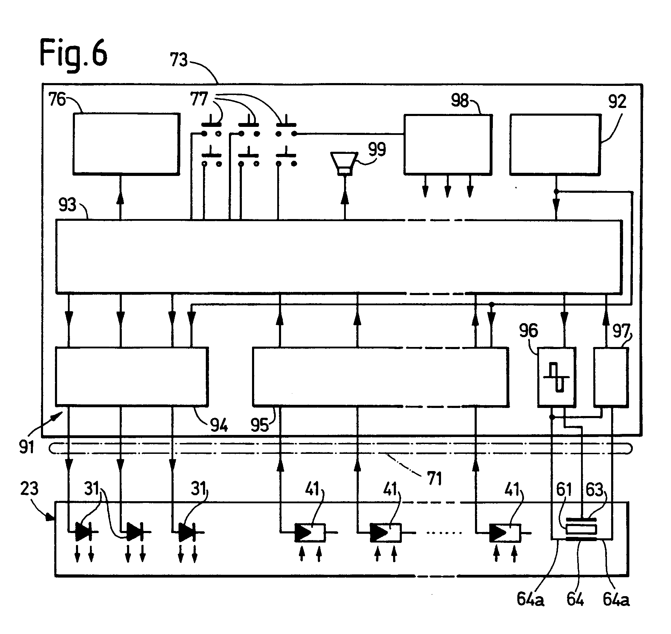

Im Gehäuse 75 der Messvorrichtung 73 sind elektronische Schaltungsmittel 91 untergebracht, deren Blockschema in der Fig. 6 ersichtlich ist. Die Schaltungsmittel 91 weisen zum Beispiel einen Taktgeber 92 und eine Auswertungs- und Steuerungsvorrichtung 93 auf, die zumindest zum Teil durch einen mit dem Taktgeber verbundenen Mikroprozessor gebildet ist. Eine Lichtquellen-Speisevorrichtung 94 hat mit dem Taktgeber 92 sowie mit der Vorrichtung 93 verbundene Eingänge und über das Kabel 71 mit den Lichtquellen 31 des Sensors 23 verbundene Ausgänge. Die Lichtempfänger 41 des Sensors 23 sind über das Kabel 71 mit Eingängen einer zu den Schaltungsmitteln 91 gehörenden Aufbereitungs- und Digitalisiervorrichtung 95 verbunden, die noch einen mit dem Taktgeber 92 verbundenen Eingang und mit der Vorrichtung 93 verbundene Ausgänge hat. Ein Ultraschallgenerator 96 hat mindestens einen mit der Auswertungs- und Steuerungsvorrichtung 93 verbundenen Eingang und über das Kabel 71 mit der Elektrode 63 sowie mit dem einen Anschluss 64a der Elektrode 64 verbundene Ausgänge. Dieser Anschluss 64a und der andere Anschluss 64a der auch als Temperaturfühler dienenden Elektrode 64 sind ferner mit einer Temperaturmessvorrichtung 97 verbunden, die ihrerseits noch mit der Auswertungs- und Steuerungsvorrichtung 93 verbunden ist. Eines der manuell betätigbaren Schalt- und/oder Stellorgane 77 dient als Ein- und Ausschalter und ist mit einer Spannungsversorgungsvorrichtung 98 verbunden, welche die restlichen Vorrichtungen der Schaltungsmittel mit den für ihren Betrieb erforderlichen Spannungen versorgt. Die übrigen Schalt- und/oder Stellorgane 77 sind mindestens zum Teil mit der Auswertungs- und Steuerungsvorrichtung 93 verbunden. Ferner kann noch ein mit der Vorrichtung 93 verbundener, akustischer Alarmgeben 99 vorhanden sein.

Nun soll der Betrieb der Einrichtung 21 erläutert werden. Hierbei und angenommen, dass die Einrichtung geeicht ist und dass der Sensor 23 gemäss der Fig. 1 an der Oberfläche des Körper 1 anliegt sowie mit Klebstreifen oder in anderer Weise lösbar an diesem befestigt ist. Zwischen dem Sensor 23 und der Hautoberfläche ist noch die bereits erwähnte, dünne, aus gallertartigem Polyäthylenglykol bestehende Ultraschallübertragungsschicht, aber kein Luft enthaltender Zwischenraum vorhanden.

Wenn die Messvorrichtung 73 eingeschaltet ist, steuert die Auswertungs- und Steuerungsvorrichtung 93 die Lichtquellen-Speisevorrichtung 94 derart, dass diese den drei Lichtquellen 31 zeitlich gegeneinander verschobene Impulsfolgen zuführt, so dass die drei Lichtquellen 31 zyklisch Lichtimpulse erzeugen. Die Frequenz dieser Impulsfolgen ist wesentlich grösser als die Puls- oder Herzschlagfrequenz und beträgt mindestens etwa 100 Hz und zum Beispiel 1 kHz bis 5 kHz. Die einzelnen Lichtimpulse sollen dabei eine weniger als einen Drittel der Perioden- oder Zyklusdauer betragende Dauer haben und derart zeitlich verteilt sein, dass in jedem Zeitpunkt nur Licht einer einzigen Wellenlänge erzeugt wird. Die erzeugten Lichtimpulse werden dann durch die Lichtleitmittel 33 hindurch bei den Lichtstrahlungsstellen 34 aus dem Sensor 23 heraus und in die Haut 3 des Körper 1 hinein gestrahlt.

Die Strömung des Blutes, welches durch eine in der mittleren oder tieferen Epidermis liegende Arterie 13 strömt, ist ausgeprägt pulsierend, wobei die Pulsation selbstverständlich im Takt der Herzschläge oder des Pulses erfolgt. Dagegen ist die Blutströmung in den sich in der äusseren oder oberen Dermis befindenden Arteriolen 9 höchstens wenig pulsierend und in den Kapillarschleifen 11, in den Venolen 10 sowie in den Venen 14 praktisch gleichmässig. Das in die Haut 3 eingestrahlte, die angegebenen Wellenlängen aufweisende Licht kann bis in die mittlere und/oder tiefere Schicht der Dermis 6 und insbesondere auch in die in dieser vorhandenen Blutgefässe eindringen. Wie in der Einleitung erörtert, durchdringt das Licht im Blut rote Blutkörperchen sowie andere Blutzellen und wird dabei durch Mehrfach-Streuung - nämlich vor allem durch Brechungsvorgänge - umgelenkt. Die roten Blutkörperchen absorbieren einen Teil des Lichtes, wobei die Absorption von der Wellenlänge des Lichtes und davon abhängig ist, ob das Hämoglobin der Blutkörperchen Sauerstoff enthält oder nicht. Ein Teil des durch Blutkörperchen umgelenkten Lichtes kann dann zur Oberfläche der Haut 3 zurückgestreut werden und bei den Lichteinlassfelden 44 bzw. Lichtempfangsbereiche 45 in die Lichtleitmittel hinein und durch diese hindurch in die Lichtempfänger 41 gelangen.

Die Intensität des Lichtes, welches von Blut gestreut wird, das pulsierend durch in der mittleren oder tieferen Schicht der Dermis liegende Arterien 13 strömt, ist im Takt der Puls- bzw. Herzschlagfrequenz pulsierend. Dies ist für das Licht von allen drei Lichtarten, d.h. das Licht mit allen drei Wellenlängen der Fall. Wie schon in der Einführung beschrieben, wird das in die Haut 3 eingestrahlte Licht jedoch nicht nur durch das pulsierend durch mindestens eine Arterie 13 strömende Blut gestreut. Das Licht wird nämlich bereits in der Epidermis 5, insbesondere in der aus abgestorbenen Zellen bestehenden Hornhaut gestreut. In der Dermis 6 verursachen auch das Gewebe, die Haarfollikel, Schweissdrüsen, Nerven, das nur wenig pulsierende, die Arteriolen 9 durchströmende Blut und das praktisch überhaupt nicht pulsierende, Kapillarschleifen 11, Venolen 10 und Venen 14 durchströmende Blut eine Streuung des eingestrahlten Lichtes. Es kann daher auch gestreutes Licht, dessen Intensität nicht pulsiert, zu Lichteinlassfeldern 44 bzw. Lichtempfangsbereichen 45 und in die Lichtempfänger 41 gelangen.

Die Lichtempfänger 41 wandeln die in sie gelangenden Lichtimpulse in elektrische Signale, nämlich in Spannungsimpulse um, deren Höhe ein Mass für die Intensität des in die Lichtempfänger gelangten Lichtes gibt. Die von den Lichtempfängern erzeugten, elektrischen Signale, d.h. Spannungsimpulse, werden dann der Aufbereitungs- und Digitalisiervorrichtung 95 zugeführt. Diese erzeugt bei jedem Lichtimpuls ein elektrisches Signal, das in digitaler Form ein Mass für die Lichtintensität gibt. Die von der Aufbereitungs- und Digitalisiervorrichtung 95 erzeugten Signale werden dann der Auswertungs- und Steuerungsvorrichtung 93 zugeführt.

Das in die Lichtempfänger 41 gelangende Licht enthält einen nicht pulsierenden, d.h. zeitlich konstanten Teil, einen im Takt der Puls- bzw. Herzschlagfrequenz periodisch ändernden Teil und noch einen unregelmässig, d.h. mehr oder weniger stochastisch und nicht periodisch ändernden Teil, der üblicherweise als physiologisches Rauschen bezeichnet wird. Für die Bestimmung der Sauerstoffsättigung und anderer interessierender Grössen werden die im Takt der Puls- bzw. Herzschlagfrequenz pulsierenden Teile der die drei verschiedenen Wellenlängen aufweisenden Lichtarten verwendet. Die Auswertungs- und Steuerungsvorrichtung 93 ist ausgebildet, um die von jedem Lichtempfänger empfangenen Lichtimpulse aufgrund ihres zeitlichen Eintreffens den drei Wellenlängen zuzuordnen. Die Vorrichtung 93 ist des weiteren derart ausgebildet, dass sie beim Betrieb in Zusammenwirkung mit der Aufbereitungs- und Digitalisiervorrichtung 95 auch die Pulsperiodendauer sowie die Pulsfrequenz ermittelt und die Funktion eines digital arbeitenden, kumulativen Filters ausübt. Für die kumulative Filterung kann die Vorrichtung 93 zum Beispiel jede Pulsperiodendauer in eine Anzahl gleich langer Zeitintervalle unterteilen. Die Aufteilung in Zeitintervalle wird dabei vorzugsweise durch das am wenigsten vom Rauschen beeinflusste Intensitätsmaximum getriggert. Die Vorrichtung 93 weist ferner einen Speicher auf, in welchem für jeden Lichtempfänger jedem der genannten Intervalle ein Speicherplatz zugeordnet ist. Die Messwerte können dann während einer vorgegebenen Anzahl von aufeinanderfolgenden Pulsperioden für jeden Lichtempfänger 41 separat in den Speicherplätzen gespeichert und bei jeder Pulsperiode kumuliert, d.h. addiert werden.

Da der periodisch ändernde Teil der Lichtintensität in einem bestimmten Zeitintervall bei jeder Messung den gleichen Wert hat, während der Wert des in das betreffende Zeitintervall fallenden Rauschsignals von Periode zu Periode stochastisch ändert, wird das Verhältnis zwischen dem Wert des periodisch ändernden Signals und dem Wert des Rauschsignals durch das Kumulieren vergrössert. Die Kumulation kann abhängig vom Rauschanteil und von der gewünschten Messgenauigkeit zum Beispiel während etwa fünf bis hundert Herzschlägen durchgeführt werden.

Wenn der Kumulationsvorgang für die vorgesehene Anzahl Perioden durchgeführt wurde, stellen die gespeicherten Werte für jeden Lichtempfänger 43 drei Treppenkurven dar. Jede von diesen stellt ihrerseits den sich über eine Periodendauer ersteckenden, zeitlichen Verlauf der Intensität von einer Lichtart dar, die eine der drei Wellenlängen aufweist. Die Auswertungs- und Steuerungsvorrichtung 93 bestimmt ferner für jede dieser Treppenkurven den Hub der periodisch ändernden, kumulierten Lichtintensität, d.h. die Differenz zwischen dem Maximalwert und dem Minimalwert der durch eine Treppenkurve dargestellten, kumulierten Lichtintensität. Wenn n Kumulationen durchgeführt wurden, ist die genannte Differenz gleich der 2n-fachen Amplitude des periodisch ändernden Teils der Lichtintensität und gibt also ein Mass für die Amplitude dieses periodisch ändernden Teils der Lichtintensität.

Die Auswertungs- und Steuerungsvorrichtung 93 kann aus den Hüben bzw. Amplituden des Lichtes mit einer Wellenlänge von 800 bis 830 nm und des Lichtes mit einer Wellenlänge von ungefähr 660 nm die Sauerstoffsättigung ermitteln.

Zur Erläuterung der Ermittlung der Sauerstoffsättigung durch die erfindungsgemässe Einrichtung werden nun noch einige Einzelheiten der in der Haut stattfindenden Streusowie Absorptionsvorgänge und des Messvorgangs näher beschrieben. Die von den Lichtquellen 31 erzeugten und die Lichtleitmittel 33 passierenden Lichtstrahlenbündel haben beim Austritt aus dem Sensor mindestens annähernd zur Hautoberfläche rechtwinklige Strahlungsmittelachsen. Die Lichtleitmittel 33 gewährleisten zumindest weitgehend, dass von den Lichtquellen 31 erzeugtes Licht nicht ohne mindestens einen im Innern der Haut erfolgenden Streuvorgang in zur Epidermis 5 oder zur Dermis 6 gehörende Hautbereiche gelangen kann, die sich in zur Hautoberfläche rechtwinkliger Blickrichtung gerade vor einem der Lichtempfangsbereiche 45 befinden. Hierzu ist in bezug auf die Fig. 1 noch anzumerken, dass in dieser die Haut 3 im Vergleich zu den Abständen der Lichtempfänger 41 von den Lichtquellen 31 mit sehr stark übertriebener Dicke gezeichnet wurde. Die zwischen den Lichtquellen 31 und der Haut 3 angeordneten Lichtleitmittel 33 isolieren die Haut 3 elektrisch und auch in einem gewissen Mass thermisch gegen die Lichtquellen 31, so dass die Haut durch die von den Leuchtdioden erzeugte Wärme nicht beschädigt wird.

Wie in der Einleitung beschrieben, hat die Intensität des Lichtes, das in der mittleren und/oder tieferen Schicht der Dermis von pulsierend durch mindestens eine Arterie 13 strömendem Blut gestreut wird und wieder aus der Haut austritt, ein Maximum, in einem gewissen Abstand vom Lichtstrahlungsbereich 35. Dieser Abstand ist von anatomischen Eigenschaften - wie der Dicke und dem Aufbau der verschiedenen Hautschichten, der Tiefe der Arterien 13 sowie dem Hämatocrit-Wert- und also von individuellen Eigenschaften der untersuchten Person sowie der gewählten Mess-Stelle abhängig. Des weitern ist der Abstand von zeitlich veränderbaren Grössen, wie dem bereits erwähnten Hämatocrit-Wert und der Durchblutung abhängig, wobei die letztere unter anderem durch die in den Wänden der Arterien 13 vorhandenen Muskelfasern und durch die Anastomosen 15 beeinflusst werden kann.

Das zur Hautoberfläche zurückgestreute Licht kann in verschiedenen Richtungen aus der Haut austreten. Die vor den Lichtempfängern 41 angeordneten, die Lichteinlassfelder 44 und Lichtempfangsbereiche 45 bildenden Lichtleitmittel 43 lassen vom Streulicht, dessen Richtungen parallel zur Schnittebene der Fig. 1 sind, nur dasjenige zu den Lichtempfängern gelangen, das mit einer zur Hautoberfläche senkrechten Geraden einen Winkel bildet, der höchstens gleich dem gewählten Winkel-Grenzwert alpha und also zum Beispiel höchstens 20° ist. Die Lichtleitmittel 43 tragen daher dazu bei, dass vor allem in relativ tiefen Schichten der Haut 3 gestreutes Licht zu den Lichtempfängern 41 gelangt. Die Lichtleitmittel 43 isolieren ferner die Haut 3 elektrisch und in einem gewissen Mass auch thermisch gegen die Lichtempfänger 41.

Der Mikroprozessor der Auswertungs- und Steuervorrichtung 93 ist derart ausgebildet und programmiert, dass er den Lichtempfänger ermittelt, bei dem die Lichtintensitäten ein vorgegebenes Auswahl-Kriterium erfüllen. Der Mikroprozessor kann zum Beispiel den Lichtempfänger ermitteln, bei welchem die Amplitude des periodisch ändernden Teils des Lichtes mit der 800 bis 830 nm betragenden und also in der Nähe der isobestischen Wellenlänge liegenden Wellenlänge ein Maximum hat. Der Mikroprozessor verwertet dann ausschliesslich die von diesem Lichtempfänger gemessenen und durch elektrische Signale dargestellten, im Takt der Pulsfrequenz pulsierenden Lichtintensitäten für die Ermittlung der Sauerstoffsättigung. Der Mikroprozessor wählt also das sich vor dem betreffenden Lichtempfänger befindende Lichteinlassfeld 44 bzw. den mit diesem identischen Lichtempfangsbereich 45 als Verwertungsbereich aus und verwertet nur das bei diesem in den Sensor gelangende Licht zum Messen der Sauerstoffsättigung.

Gemäss der Einleitung könnten jedoch stattdessen die Signale von demjenigen Lichtempfänger für die Bestimmung der Sauerstoffsättigung verwertet werden, bei welchem das Verhältnis zwischen der genannten Amplitude und dem Maximalwert oder dem Mittelwert oder dem Minimalwert des gesamten, ungefähr die isobestische Wellenlänge aufweisenden Lichtes ein Maximum hat. Es sei angemerkt, dass normalerweise all diese verschiedenen Auswahl-Kriterien für ein und denselben Lichtempfänger erfüllt sind.

Unter Umständen kann sich die günstigste Lichtempfangsstelle ungefähr in der Mitte zwischen zwei einander benachbarten Lichteinlassfeldern 44 bzw. Lichtempfangsbereichen 45 befinden, so dass das vorgegebene Auswahl-Kriterium für zwei Lichteinlassfelder 44 ungefähr gleich gut erfüllt ist. Möglicherweise kann es sogar geschehen, dass sich bei mehr als zwei, beispielsweise drei Lichteinlassferldern 44 ungefähr optimale Messbedingungen ergeben. Der Mikroprozessor kann nun eventuell derart ausgebildet und programmiert sein, dass er in einem solchen Fall für jede Lichtart, d.h. Wellenlänge, die von zwei oder noch mehr Lichtempfängern gemessenen Lichtintensitäten addiert und für die Bestimmung der Sauerstoffsättigung verwertet. Das bedeutet, dass für die Bestimmung der Sauerstoffsättigung ein Verwertungsbereich ausgewählt oder bestimmt wird, der zwei oder eventuell noch mehr entlang der Geraden 47 aufeinanderfolgende Lichteinlassfelder 44 bzw. Lichtempfangsbereiche 45 enthält. Die Grösse des Verwertungsbereichs, bei dem das in den Sensor gelangende Licht zum Messen der Sättigung verwertet wird, kann also gewissermassen elektronisch verändert werden. Dabei wird jedoch normalerweise immer nur das durch einen Teil der Lichteinlassfelder in den Sensor gelangende Licht und nie das durch alle Lichteinlassfelder 44 in den Sensor gelangende Licht für die Bestimmung der Sauerstoffsättigung verwertet.

Durch die mit den Lichtleitmitteln 33, 43 erzielte Elimination von unerwünschtem Streulicht und durch die in der beschriebenen Weise erfolgende Auswahl mindestens eines günstigen Lichtempfängers 41 und mindestens eines günstigen, diesem zugeordneten Lichtempfangsbereich für die Ermittlung der Sauerstoffsättigung kann erreicht werden, dass bei allen Messungen viel zeitlich periodisch im Takt der Puls- bzw. Herzschlagfrequenz änderndes, von arteriellem Blut gestreutes Licht in den gewählten Lichtempfänger gelangt. Dies wiederum gewährleistet eine hohe Messgenauigkeit.

Die Ultraschallquelle 61 erzeugt während der Messvorgänge und eventuell schon vor dem eigentlichen Messbeginn impulsweise Ultraschallwellen. Ein Teil der Wellenenergie wird durch den Ultraschallübertragungs- und Heizkörper 67 absorbiert und in Wärme umgewandelt. Der in Wärme umgewandelte Energieteil beträgt vorzugsweise etwa 30% bis 70% der gesamten, dem Ultraschallübertragungs- und Heizkörper 67 in Form von Ultraschallwellen zugeführten Energie. Die in Wärme umgewandelte Energie erwärmt den Ultraschallübertragungs- und Heizkörper 67 auf die bereits angegebene Temperatur, die über der normalerweise, an der Hautoberfläche vorhandenen Temperatur liegen. Die Temperatur des Ultraschallübertragungs- und Heizkörpers 67 wird durch die auch als Temperaturfühler dienende Elektrode 64 in Zusammenwirkung mit der Temperaturmessvorrichtung 97 ermittelt. Diese führt den Mikroprozessor der Auswertungs- und Steuerungsvorrichtung 93 ein den Wert der Temperatur darstellendes Digitalsignal zu. Die Vorrichtung 93 regelt den Ultraschallgenerator 96 derart, dass die gemessene Temperatur auf einem vorgegebenen Sollwert gehalten wird. Die Regelung der Temperatur kann zum Beispiel durch Impulsbreitenregelung der erzeugten Ultraschallwellen-Impulse erfolgen.

Der erwärmte Ultraschallübertragungs- und Heizkörper 67 führt der Haut 3 durch Wärmeleitung Wärme zu. Ferner werden auch Ultraschallwellen auf die Haut übertragen und in dieser absorbiert. Diese Absorption erfolgt vor allem in den durchbluteten, lebende Zellen enthaltenden Hautschichten, so dass durch die Ultraschallwellen direkt innere Schichten der Haut erwärmt werden. Dabei erfolgt sowohl die Wärmeleitung als auch die Ultraschallwellenübertragung zum Teil durch die Lichtquellen, Lichtempfänger und Lichtleitmittel hindurch. Übrigens kann die in den Lichtquellen und Lichtempfängern aus elektrischer Energie entstehende Wärme ebenfalls noch zur Erwärmung der Haut beitragen. Die kontinuierliche Erwärmung der Haut durch Wärmeleitung und durch im Innern der Haut absorbierte Ultraschallwellen ermöglicht, den zur Messung der Sauerstoffsättigung benutzten Hautbereich auf eine relativ hohe, ziemlich gleichmässige, die Durchblutung verstärkende Temperatur zu erwärmen, ohne dass irgend ein Bereich der Haut durch Überhitzung beschädigt wird. Da sich der Block, der aus dem Ultraschallübertragungs- und Heizkörper 67 und den in diesen eingebetteten Bauteilen besteht, bis zu der vom Gehäuserand 25a definierten Ebene erstreckt, muss zum Messen ausser der erwähnten, dünnen, zwischen dem Sensor und der Haut anzubringenden Schicht aus Polyähtylenglykol kein solches und auch kein anderes, zur Ultraschallübertragung dienendes, halbfestes sowie einigermassen fliessfähiges oder flüssiges Material in irgendwelche Hohlräume eingebracht werden. Die Benutzung des Sensors 23 ist daher sehr einfach und insbesondere einfacher als die Benutzung der in der Einleitung kommentierten, aus der US-A-4 890 619 bekannten Sensoren.

Die dritte Lichtart, d.h. das von der dritten Lichtquelle 31 erzeugte, eine ungefähr 575 nm betragende Wellenlänge aufweisende Licht wird durch sauerstoffloses, sauerstoffhaltiges und kohlenmonoxydhaltiges Hämoglobin relativ stark absorbiert. Ferner haben die Licht-Absorptionskoeffizienten von sauerstofflosem und sauerstoffhaltigem Hämoglobin bei dieser Wellenlänge ziemlich ähnliche Grössen. Das Verhältnis zwischen der Amplitude der Intensität des zeitlich periodisch im Takt der Pulsfrequenz ändernden Streulichts der dritten Lichtart und dem Maximalwert oder Mittelwert oder Minimalwert der Intensität des gesamten Streulichts der dritten Lichtart gibt daher ein von der Gassättigung des Hämoglobins weitgehend unabhängiges Mass für die relative Grösse des Volumens des Blutes, das in demjenigen Hautabschnitt vorhanden ist, in den das Licht eindringt. Die Auswertungs- und Steuervorrichtung 93 ist ausgebildet, um das letztgenannte Verhältnis zu ermitteln. Eventuell kann die Vorrichtung 93 aus dem genannten Verhältnis aufgrund von durch Modellrechnungen ermittelten Formeln und/oder von tabellarisch gespeicherten Wert-Zuordnungen sowie Eichungen absolute Werte für das Verhältnis zwischen dem Blutvolumen und dem gesamten Volumen des erfassten Hautabschnitts bestimmen. Die Ermittlung der ein Mass für das Blutvolumen gebenden Grösse kann wahlweise mit oder ohne Erwärmung der Haut erfolgen.

Im übrigen wird vorzugsweise auch vom Licht der dritten Lichtart nur dasjenige für die Bestimmung des Blutvolumens verwertet, welches durch den für die Bestimmung der Sauerstoffsättigung gewählten Verwertungsbereich in den Sensor gelangt.

Die Anzeigevorrichtung 76 zeigt den mit SA bezeichneten Wert der Sauerstoffsättigung, die mit P bezeichnete Pulsfrequenz und einen mit BV bezeichneten, ein Mass für das Blutvolumen gebenden Wert an.

Die Schalt- und/oder Stellorgane 77 ermöglichen, mindestens einen oberen und/oder unteren Grenzwert für die Sauerstoffsättigung und eventuell für andere gemessene Grössen einzugeben. Das Über- oder Unterschreiten eines solchen Grenzwerts kann dann optisch durch die Anzeigevorrichtung 76 und/oder akustisch durch den akustischen Alarmgeber 99 signalisiert werden.

Die Einrichtung 21 wird vom Hersteller vor der Auslieferung geeicht. Unter Umständen können sich jedoch die Intensitäten der von den drei Lichtquellen erzeugten Lichtimpulse oder die Empfindlichkeiten der verschiedenen Lichtempfänger für die verschiedenen Lichtwellenlängen ändern. Unter der Empfindlichkeit wird hierbei das Verhältnis zwischen der Höhe des von einem Lichtempfänger erzeugten, elektrischen Spannungsimpulses und der Intensität eines in den Lichtempfänger gelangenden Lichtimpulses verstanden.

Wenn der Sensor 23 nicht zum Messen benutzt wird, kann er vorübergehend in der Vertiefung 82a des Supports 82 des Eich-Lichtverteilers 81 gehalten sein. Die Lichtstrahlungsstellen 35 und Lichtempfangsbereiche 45 des Sensors 23 sind dann den Lichtkopplungselementen 85 bzw. 86 des Eich-Lichtverteilers 81 zugewandt und liegen beispielsweise an diesen an. Ein Benutzer kann dann die elektronischen Schaltungsmittel durch manuelles Betätigen von mindestens einem der Schalt- und/oder Stellorgane 77 in einen Eichmodus bringen. In diesem steuert die Auswertungs- und Steuerungsvorrichtung 93 die Lichtquellen-Speisevorrichtung 94 derart, dass jede der drei Lichtquellen 31 mindestens einen Lichtimpuls erzeugt. Ein Teil des bei den Lichtstrahlungsstellen 34 aus dem Sensor herausgestrahlten Lichtes wird dann durch den Eich-Lichtverteiler 81 auf die verschiedenen Lichteinlassflächen 44 bzw. Lichtempfangsbereiche 45 des Sensors verteilt und dort dessen Lichtempfängern 41 zugeführt. Jeder Lichtempfänger erzeugt dann für jede der drei Lichtarten einen Spannungsimpuls. Die Höhen - d.h. Spannungswerte - dieser Impulse und/oder vom Mikroprozessor daraus abgeleiteten Werte wurden dann als Eichwerte in Speicherplätzen eines zur Auswertungs- und Steuerungsvorrichtung 93 gehörenden Speichers bis zur nächsten Eichung gespeichert. Nach dem Eichen kann die Einrichtung durch manuelles Betätigen von mindestens einem der Schalt- und /oder Stellorgane 77 oder durch ein durch das Entfernen des Sensors 23 vom Support 82 betätigtes Schaltorgan in den normalen Betriebsmodus gebracht werden. In diesem werden die neu ermittelten und gespeicherten Eichwerte dann zur Verwertung der gemessenen Lichtintensitätswerte herangezogen. Durch derartige Eichungen kann gewährleistet werden, dass die Einrichtung 21 auch nach langdauerndem Gebrauch noch genaue Messresultate liefert.

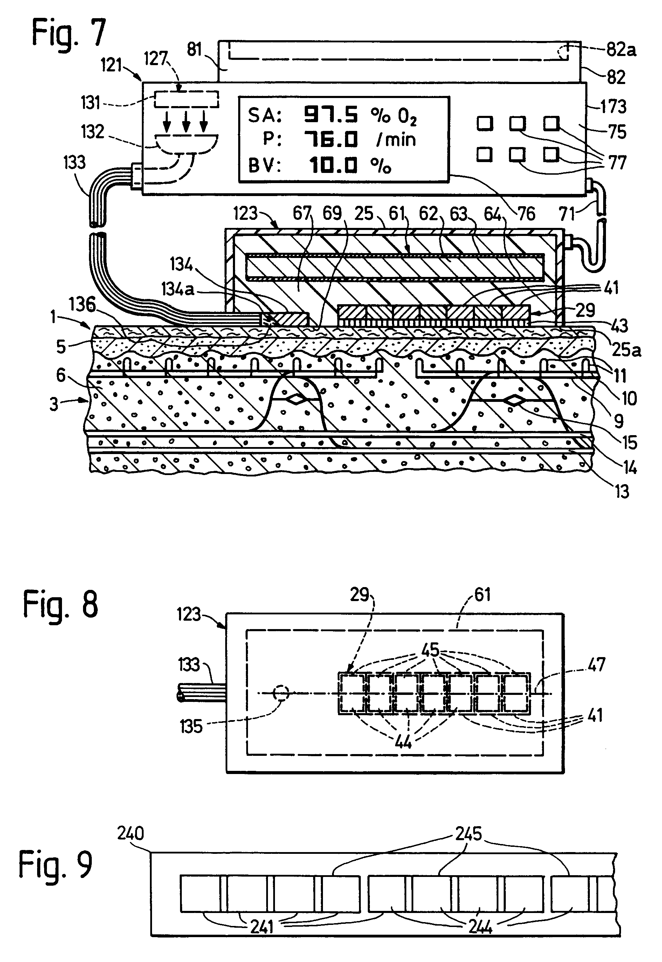

Die in der Fig. 7 ersichtliche Einrichtung 121 besitzt einen zum Teil dem Sensor 23 entsprechenden Sensor 123 sowie eine zum Teil der Messvorrichtung 73 entsprechende Messvorrichtung 173. Soweit nachfolgend nichts anderes angegeben wird, können die beiden Einrichtungen ähnlich ausgebildet sein, wobei einander entsprechende Teile mit identischen Bezugszeichen bezeichnet sind. Die Einrichtung 121 besitzt jedoch Lichtstrahlungsmittel 127, die anders ausgebildet sind als die Lichtstrahlungsmittel 27 der Einrichtung 21. Die Lichtstrahlungsmittel 127 besitzen zur Erzeugung von Licht der verschiedenen benötigten Lichtarten mindestens eine ausserhalb des Sensors 123 im Gehäuse 75 der Messvorrichtung 173 angeordnete Lichtquelle 131. Die Lichtstrahlungsmittel 127 können zum Beispiel analog wie die Lichtstrahlungsmittel 27 mehrere je aus einer Halbleiterdiode bestehende Lichtquellen aufweisen, von denen jede Licht mit einer anderen Wellenlänge erzeugt. Wenn die bzw. jede Lichtquelle 131 beim Betrieb Licht mit verschiedenen Wellenlängen - d.h. mit einem breiten Spektrum - erzeugt, können die Lichtstrahlungsmittel ferner mindestens ein Interferenzfilter aufweisen, das aus dem erzeugten Lichtspektrum dasjenige Licht ausfiltert und durchlässt, das für jede Lichtart genau die gewünschte Wellenlänge aufweist.

Die Messvorrichtung 173 besitzt ferner einen Lichtkollektor 132, um das von der mindestens einen Lichtquelle 131 erzeugte Licht zu sammeln und dem einen, im und/oder am Gehäuse 75 gehaltenen Ende von länglichen, flexiblen Lichtleitmitteln 133 zuzuführen, die mindestens eine Glasfaser besitzen. Das andere Ende der Lichtleitmittel 133 ist mit dem Sensor 123 verbunden. Dieser kann zum Beispiel auf seiner beim Messen am Körper 1 anliegenden Seite eine Nut aufweisen, in welcher das letztgenannte Ende der Lichtleitmittel 133 und ein metallischer Reflektor 134 angeordnet sind, der eine 45° gegen die am Körper 1 anliegende Sensorseite geneigte, dem Ende der Lichtleitmittel 133 zugewandte Reflektorfläche 134a besitzt. Die Lichtleitmittel 133 definieren zusammen mit dem Reflektor 134 einen in der Fig. 8 ersichtlichen Lichtstrahlungsbereich 135, bei welchem das Licht aus dem Sensor 123 heraus und in den Körper 1 gestrahlt werden kann. Der zwischen dem im Sensor befestigten Ende der Lichtleitmittel 133 und dem Reflektor 134 vorhandende Zwischenraum ist zum Beispiel mit einem lichtdurchlässigen Material 136 ausgefüllt. Der Lichtkollektor 132, die Lichtleitmittel 133, der Reflektor 134 und das Material 136 bilden zusammen Lichtübertragungsmittel, mit denen das von der mindestens einen Lichtquelle 131 erzeugte Licht zum Lichtstrahlungsbereich 135 übertragen werden kann.

Beim Betrieb der Einrichtung 121 kann das Licht aller erzeugten Lichtarten sequentiell bei ein und derselben Lichtstrahlungsstelle - d.h. durch ein und dieselbe Fläche - des Lichtstrahlungsbereichs 135 aus dem Sensor 123 heraus gestrahlt werden. Der Lichtstrahlungsbereich 135 kann in einer Draufsicht auf die beim Messen am Körper 1 anliegende Seite des Sensors beispielsweise kreisförmig sein und in der genannten Draufsicht in allen Richtungen eine sehr kleine, vorzugsweise höchstens 2 mm, beispielsweise höchstens 1 mm oder sogar nur höchstens 0,5 mm betragende Ausdehnung haben. Im übrigen haben die Lichtleitmittel 133 ähnlich wie die Lichtleitmittel 33 eine die Lichtstrahlen mehr oder weniger parallel richtende und kollimierende Wirkung.

Die aus separaten Fotohalbleitern bestehenden Licht-emfpänger 41 der Einrichtungen 21 und 121 können durch eine integrierte Schaltung ersetzt werden, wie sie schematisch in der Fig. 9 dargestellt und mit 240 bezeichnet ist. Die integrierte Schaltung 240 besitzt eine gerade Reihe von Fotohalbleitern, nämlich Fotodioden, von denen jede einen Lichtempfänger 241 bildet. Der die integrierte Schaltung 240 enthaltende Sensor kann zum Beispiel noch in der Fig. 9 nicht gezeichnete Lichtleitmittel 43 besitzen, die analog angeordnet sind wie bei den Einrichtungen 21 und 121. Jeder Lichtempfänger 241 deniniert bei der beim Messen am zu untersuchenden Körper anliegenden Seite des Sensors ein Lichteinlassfeld 244. Die Lichteinlassfelder 244 können zum Beispiel quadrat- oder rechteckförmig und durch schmale Zwischenräume getrennt sein. Die integrierte Schaltung 240 kann unter Umständen eine relativ grosse, zum Beispiel mindestens 100 betragende Anzahl Fotodioden enthalten. Der Mikroprozessor der mit der integrierten Schaltung verbundenen, elektronischen Schaltungsmittel kann daher zum Beispiel ausgebildet sein, um jeweils die von einer Gruppe in der Längsrichtung aufeinanderfolgender Lichtempfänger 241 gemessenen Lichtintensitäten vor oder nach der kumulativen Filterung zu addieren und jeder dieser Gruppen einen Lichtempfangsbereich 245 zuzuordnen. Dabei können alle Lichtempfangsbereiche 245 eine gleich grosse Anzahl von Lichteinlassfeldern 244 enthalten. Bei der Benutzung des die integrierte Schaltung 240 enthaltenden Sensors werden zuerst die Lichtintensitäten bei jedem Lichtempfangsbereich 245 separat gemessen. Danach wird analog wie bei der Einrichtung 21 beispielsweise einer der Lichtempfangsbereiche 245 als Verwertungsbereich ausgewählt und das bei diesem in den Sensor gelangende bzw. gelangte Licht für die Ermittlung der Sauerstoffsättigung verwertet. Eventuell kann jedoch der Mikroprozessor für die Ermittlung der Sauerstoffsättigung auch die von zwei oder mehr Gruppen von Lichtempfängern gemessenen Lichtintensitäten addieren und also die Grösse des Verwertungsbereichs, in welchem das Licht verwertet wird, beim Messen analog variieren, wie es bereits für die Lichtempfänger 41 erläutert wurde.

Die Einrichtungen können noch auf andere Arten ausgebaut und/oder geändert werden. Die Einrichtungen können zum Beispiel ausgebildet werden, um auch noch die Kohlenmonoxydsättigung des Blutes zu ermitteln und anzuzeigen. Die Kohlenmonoxydsättigung kann zum Beispiel aus dem Intensitätsverhältnis zwischen der dritten und der zweiten Lichtart ermittelt werden. Eventuell können für die Ermittlung der Kohlenmonoxydsättigung noch Lichtimpulse einer vierten Lichtart mit einer Wellenlänge erzeugt und gemessen werden, die kürzer ist als diejenige der ersten, zweiten und dritten Lichtart. Die Kohlenmonoxydsättigung kann dabei durch die Verwertung der Intensitäten von Licht gemessen werden, das beim gleichen Verwertungsbereich in den Sensor gelangt, wie das für die Ermittlung der Sauerstoffsättigung verwertete Licht. Der Wert der gemessenen Kohlenmonoxydsättigung kann zum Beispiel in Prozenten angezeigt werden.

Es besteht jedoch auch die Möglichkeit, Lichtstrahlungsmittel vorzusehen, die beim Betrieb nur zwei verschiedene Wellenlängen aufweisendes Licht in den Körper eines zu untersuchenden Patienten hineinstrahlen. Ferner können die Lichtquellen - insbesondere wenn sie ausserhalb des Sensors angeordnet sind - statt aus den bereits erwähnten Leucht- oder Laserdioden aus andersartigen Lasern bestehen.

Die Lichtleitmittel 33, 43 können durch Lichtleitmittel ersetzt werden, die anstelle von ebenen Glasscheiben ein Bündel von kurzen, nebeneinander angeordneten, miteinander verklebten oder in anderer Weise fest verbundenen Abschnitten von Glasfasern aufweisen, die als Lichtleiter dienen. Solche Lichtleitmittel ergeben dann eine zu den Achsen der Fasern rotationssymmetrische Kollimation. Zudem können die Lichtleitmittel 33 zusammen mit den Lichtleitmitteln 43 aus einem einzigen, zusammenhängenden, plättchenförmigen, Scheiben oder Fasern aus Glas aufweisenden Körper gebildet werden.

Die sich vor den Lichtquellen 31 befindenden Lichtleitmittel 33 können ferner durch ein einziges, beispielsweise plättchenförmiges Glasstück gebildet werden. Bei einer solchen Ausbildung der Lichtleitmittel 31 ist es vorteilhaft, die Randflächen des Glasstück zu verspiegeln und/oder den die Randflächen umschliessenden Ultraschallübertragungs- und Heizkörper 67 aus einem opaken Giessharz zu bilden.

Die Lichtempfangsmittel können eventuell mit zwei oder drei oder eventuell sogar noch mehr Reihen von nebeneinander angeordneten Lichtempfängern ausgerüstet werden. Die zu den Lichtempfangsmitteln gehörenden Lichtleitmittel können dann für eine der Lichtempfänger-Reihen gemäss der Fig. 3 angeordnete, und zur Hautoberfläche rechtwinklige, als bLichtleiter dienende Scheiben oder zur Hautoberfläche rechtwinklige Glasfasern haben. Die den andern Lichtempfängern zugeordneten Lichtleitmittel können hingegen Scheiben oder Fasern aufweisen, die in einer zur Schnittebene der Fig. 1 parallelen Schnitt- oder Projektionsebene mit der Hautoberfläche und der Geraden 47 einen von 90° verschiedenen Winkel bilden und von der Hautoberfläche weg nach links - d.h. gegen die Lichtquellen hin - geneigt sind. Wenn insgesamt drei Reihen von Lichtempfängern vorhanden sind, kann zum Beispiel der von den Scheiben oder Fasern mit der Geraden 47 gebildete Winkel in einer zur Schnittebene der Fig. 1 parallelen Schnitt- oder Projektionsebene bei einer Reihe 90°, bei einer andern Reihe ungefähr 25° bis 45° und bei der letzten Reihe ungefähr 50° bis 70° betragen. Eventuell kann auch eine Reihe von Lichtempfängern vorgesehen werden, denen Lichtleitelemente zugeordnet sind, die in einem der Fig. 1 entsprechenden Schnitt von der Hautoberfläche weg nach rechts geneigte Scheiben oder Fasern haben, wobei der mit der Geraden 47 gebildete Winkel mindestens 60° betragen sollte. Wenn die zu den Lichtempfangsmitteln gehörenden Lichtleitmittel Scheiben oder Fasern aufweisen, die mit der Geraden 47 und der Hautoberfläche verschiedene Winkel bilden, kann die Auswertungs-und Steuerungsvorrichtung 93 ausgebildet sein, um für die Ermittlung der Sauerstoffsättigung einen Verwertungsbereich mit mindestens einem Lichtempfangsbereich auszuwählen, bei dem sowohl der Abstand vom Lichtstrahlungsbereich als auch die Hauptdurchlassrichtung der Lichtleitmittel - d.h. der Winkel der Lichtleiter mit der Hautoberfläche und der Geraden 47 - am günstigsten ist.

Eventuell kann man bei der Einrichtung 21 auf Lichtleitmittel 33 und/oder bei den Einrichtungen 21, 121 auf die Lichtleitmittel 43 verzichten.

Die Lichtempfänger und die Lichtempfangsbereiche können in einer der Fig. 2 oder 8 entsprechenden, zur Hautoberfläche rechtwinkligen Projektion konzentrisch zum Zentrum des Lichtstrahlungsbereichs gebogen sein und die Form von Kreisringsektoren aufweisen, die sich alle über den gleichen Zentriwinkel erstrecken. Des weitern besteht die Möglichkeit, in einer der Fig. 2 oder 8 entsprechenden Draufsicht auf beiden Seiten des Lichtstrahlungsbereichs und bezüglich diesen spiegelsymmetrisch angeordnete Lichtempfangsbereiche und Lichtempfänger vorzusehen.

Es wäre zudem möglich, die Lichtempfänger ausserhalb des Sensors im Gehäuse 75 unterzubringen und durch flexible Lichtleiter mit den Lichteinlassfeldern bzw. Lichtempfangsbereichen des Sensors zu verbinden. Ferner wäre es eventuell sogar möglich, nur einen einzigen Lichtempfänger vorzusehen, der optisch über eine steuerbare, optische Weiche oder Schaltvorrichtung abwechselnd mit den verschiedenen Lichteinlassfeldern bzw. Lichtempfangsbereichen verbunden werden kann.

Ferner kann man eventuell vorsehen, die beim Messen am Körper 1 anliegende Seite oder Anliegefläche des Sensors statt eben in einer Schnittebene leicht konkav gebogen auszubilden, wobei der Krümmungsradius zum Beispiel mindestens oder ungefähr 50 cm betragen kann. Dies ermöglicht, dass der Sensor gut an einer konvexen Oberfläche des Körpers des zu untersuchenden Patienten anliegen kann. Die von den Lichteinlassfeldern und Lichtempfangsbereichen gebildete Reihe soll dann vorzugsweise in einer Draufsicht auf die beim Messen am zu untersuchenden Körper anliegende Seite des Sensors immer noch gerade sein. Eventuell kann der Sensor sogar ein wenig biegbar sein, so dass seine beim Messen am zu untersuchenden Körper anliegende Seite oder Fläche sich an die Körperoberfläche anpassen kann.

Möglicherweise kann die Ultraschallquelle 61 weggelassen werden.

Der Eich-Lichtverteiler kann anstelle von Lichtleitern mindestens einen Lichtreflektor und/oder ein Lichtstreuorgan aufweisen, um von den Lichtstrahlungsmitteln gegen ihn gestrahltes Licht auf die Lichteinlassfelder und Lichtempfangsbereiche des Sensors zu verteilen. Ein Lichtstreuorgan kann zum Beispiel einen Körper mit einer Matrix aus Glas aufweisen, in der eine Vielzahl kleiner, aus einem anderen Glas oder einem sonstigen Material bestehenden, eine Lichtstreuung bewirkender Partikel dispergiert sind.

## Patentansprüche

1. Einrichtung zur Messung mindestens einer Gassättigung, insbesondere der Sauerstoffsättigung, von in einem lebenden Körper (1) vorhandenem Blut, mit einem zum Anliegen am Körper (1) bestimmten Sensor (12, 123), mit Lichstrahlungsmitteln (27), um verschiedene Wellenlängen aufweisendes Licht bei mindestens einem Lichtstrahlungsbereich (35) aus dem Sensor (23, 123) heraus in den Körper (1) zu strahlen, und mit Lichtempfangsmitteln (29), wobei der Sensor (23, 123) in verschiedenen Abständen vom Lichtstrahlungsbereich (35, 135) stehende Lichtempfangsbereiche (45, 245) aufweist, bei welchen im Körper (1) zurückgestreutes Licht zu den Lichtempfangsmitteln (27) gelangen kann, wobei die letzteren ausgebildet sind, um für jeden Lichtempfangsbereich (45, 245) die Lichtintensität des bei diesem zu ihnen gelangenden Lichtes zu ermitteln, und wobei mit den Lichtempfangsmitteln (27) verbundene Schaltungsmittel (91) vorhanden sind, um aus für die verschiedenen Wellenlängen ermittelten Lichtintensitäten die mindestens eine Gassättigung zu ermitteln, dadurch gekennzeichnet, dass die Schaltungsmittel (91) ausgebildet sind, um aufgrund eines Kriteriums einen Verwertungsbereich aus den Lichtempfangsbereichen (45, 245) auszuwählen und nur beim gewählten Verwertungsbereich zu den Lichtempfangsmitteln (29) gelangendes Licht für die Ermittlung der bzw. mindestens einer Gassättigung zu verwerten.

2. Einrichtung nach Anspruch 1, dadurch gekennzeichnet, dass die Schaltungsmittel (91) ausgebildet sind, um einen zeitlich periodisch mit der Pulsfrequenz des durch den Körper (1) strömenden Blutes ändernden Teil des empfangenen Lichtes für die Ermittlung der Gassättigung zu verwerten und um den Verwertungsbereich derart zu bestimmen, dass dieser denjenigen Lichtempfangsbereich (45, 245) enthält, bei dem die Amplitude der Intensität des genannten Teils des eine der Wellenlängen aufweisenden Lichtes oder das Verhältnis zwischen dieser Amplitude und dem Maximalwert oder dem Mittelwert oder dem Minimalwert der Intensität des gesamten, beim betreffenden Lichtempfangsbereich (45, 245) empfangenen, die genannte Wellenlänge aufweisenden Lichtes am grössten ist.

3. Einrichtung nach Anspruch 1 oder 2, dadurch gekennzeichnet, dass die Lichtempfangsmittel (29) mehrere Lichtempfänger (41) zur separaten Messung der Intensitäten des in sie gelangenden Lichtes aufweisen, dass jedem Lichtempfangsbereich (45, 245) mindestens ein Lichtempfänger (41, 241) zugeordnet ist und dass die Schaltungsmittel (91) ausgebildet sind, um dem Verwertungsbereich mindestens einen und höchstens einen Teil der Lichtempfänger (41, 241) zuzuordnen, wobei die Lichtempfänger (41, 241) vorzugsweise aus Fotohalbleitern bestehen.

4. Einrichtung nach einem der Ansprüche 1 bis 3, dadurch gekennzeichnet, dass der Sensor (23, 123) mindestens drei, sowie vorzugsweise mindestens fünf Lichtempfangsbereiche (45, 245) aufweist, welche in verschiedenen Abständen vom Lichtstrahlungsbereich (35, 135) stehen.

5. Einrichtung nach Anspruch 4, dadurch gekennzeichnet, dass die Lichtempfangsbereiche (45, 245) eine Reihe bilden, die mindestens in einer Draufsicht auf die zum Anliegen am Körper (1) bestimmte Seite des Sensors (23, 123) gerade ist, dass jeder Lichtempfangsbereich (45, 245) mindestens ein Lichteinlassfeld (44, 244) besitzt, durch das Licht in die Lichtempfangsmittel (29) gelangen kann, und dass die in der Längsrichtung der Reihe gemessene Ausdehnung jedes Lichteinlassfeldes (44, 244) mehr als die Hälfte und vorzugsweise mindestens zwei Drittel des Abstandes der Mittelpunkte von einander benachbarten Lichteinlassfeldern (44, 244) beträgt.

6. Einrichtung nach Anspruch 5, dadurch gekennzeichnet, dass die parallel zur Längsrichtung der Reihe der Lichtempfangsbereiche (45, 245) gemessene Ausdehnung des Lichtstrahlungsbereichs (35, 135) höchstens 2 mm und vorzugsweise höchstens 1 mm beträgt, wobei die quer zur genannten Längsrichtung gemessene Ausdehnung des Lichtstrahlungsbereichs (35, 135) vorzugsweise höchstens 6 mm beträgt.

7. Einrichtung nach einem der Ansprüche 1 bis 6, dadurch gekennzeichnet, dass die Lichtstrahlungsmittel (27) im Sensor (23) angeordnete Lichtquellen (31) für die Erzeugung der verschiedene Wellenlängen aufweisenden Lichtarten aufweisen, wobei die Lichtquellen (31) vorzugsweise aus Halbleiterdioden bestehen.

8. Einrichtung nach einem der Ansprüche 1 bis 6, dadurch gekennzeichnet, dass die Lichtstrahlungsmittel (127) zur Erzeugung von Licht verschiedener Wellenlängen mindestens eine ausserhalb des Sensors (123) angeordnete Lichtquelle (131) aufweisen, die durch Lichtübertragungsmittel (132, 133, 134, 136) derart mit dem Sensor (123) verbunden ist, dass die verschiedene Wellenlängen aufweisenden Arten von Licht sequentiell durch ein und dieselbe Oberfläche des Lichtstrahlungsbereichs (135) aus dem Sensor (123) heraus gestrahlt werden können.

9. Einrichtung nach Anspruch 7 oder 8, dadurch gekennzeichnet, dass die Lichtstrahlungsmittel (27, 127) zwischen der bzw. jeder Lichtquelle (31, 131) und dem Lichtstrahlungsbereich (35) angeordnete Lichtleitmittel (33, 133) aufweisen.

10. Einrichtung nach einem der Ansprüche 1 bis 9, dadurch gekennzeichnet, dass die Lichtempfangsmittel (29) zwischen den Lichtempfangsbereichen (45, 245) und den Lichtempfängern (41, 241) angeordnete Lichtleitmittel (43) aufweisen.

11. Einrichtung nach Anspruch 9 oder 10, dadurch gekennzeichnet, dass die Mittelpunkte des Lichtstrahlungsbereichs (35, 135) und der Lichtempfangsbereiche (45, 245) auf einer Mittellinie (47) liegen, die mindestens in einer Draufsicht auf die zum Anliegen am Körper (1) bestimmte Seite des Sensors (23, 123) gerade ist, und dass die Lichtleitmittel (33, 43) aus im Sensor (23, 123) angeordneten, ebenen Scheiben (51) gebildete Lichtleiter aufweisen, die in der genannten Draufsicht rechtwinklig zur Mittellinie (47) sind.

12. Einrichtung nach einem der Ansprüche 1 bis 11, dadurch gekennzeichnet, dass die Schaltungsmittel (91) ausgebildet sind, um die Pulsperiodendauer in mehrere Zeitintervalle zu unterteilen und während mehreren Pulsperioden die Intensitäten des von den Lichtempfangsmitteln (27) während der verschiedenen Zeitintervalle empfangenen Lichtes für die verschiedenen Wellenlängen separat zu kumulieren.

13. Einrichtung nach einem der Ansprüche 1 bis 12, dadurch gekennzeichnet, dass der Sensor mindestens eine Ultraschallquelle (61) und Ultraschallübertragungsmittel aufweist, um von der Ultraschallquelle (81) erzeugte Ultraschallwellen auf den lebenden Körper (1) zu übertragen, dass die Ultraschallübertragungsmittel einen Ultraschallübertragungskörper (67) aufweisen, der eine zum Anliegen am lebenden Körper (1) bestimmte Anliegefläche (69) bildet und den Lichtstrahlungsbereich (35, 135) sowie alle Lichtempfangsbereiche (45, 245) mindestens im wesentlichen umschliesst.

14. Einrichtung nach Anspruch 13, dadurch gekennzeichnet, dass der Ultraschallübertragungskörper (67) ausgebildet ist, um 30% bis 70% der ihm in Form von Ultraschallwellen zugeführten Energie in Wärme umzuwandeln und mindestens teilweise durch Wärmeleitung auf den lebenden Körper (1) zu übertragen.

15. Einrichtung nach Anspruch 13 oder 14, dadurch gekennzeichnet, dass die Ultraschallquelle (61) eine zur Erzeugung der Ultraschallwellen dienende Elektrode (64) aufweist, die durch einen auch als Temperaturfühler dienenden, elektrischen Widerstand gebildet ist.

16. Einrichtung nach einem der Ansprüche 1 bis 15, gekennzeichnet durch einen Eich-Lichtverteiler (81), der zum Eichen der Einrichtung dem Lichtstrahlungsbereich (35, 135) sowie den Lichtempfangsbereichen (45, 245) zugewandt werden kann und ausgebildet ist, um ihm von den Lichtstrahlungsmitteln (27, 127) beim Lichtstrahlungsbereich (35, 135) zugeführtes Licht auf die verschiedenen Lichtempfangsbereiche (45, 245) zu verteilen und bei diesen den Lichtempfangsmitteln (29) zuzuführen.

17. Einrichtung nach einem der Ansprüche 1 bis 16, dadurch gekennzeichnet, dass die Lichtstrahlungsmittel (27, 127) ausgebildet sind, um mindestens drei Arten von monochromatischem, je eine andere Wellenlänge aufweisendem Licht zu erzeugen, und dass eine der Lichtarten eine Wellenlänge von 500 nm bis 600 nm hat und zur Ermittlung einer Grösse dient, die ein Mass für das Volumen des in einem Abschnitt des lebenden Körpers (1) vorhandenen Blutes gibt.

## Claims

1. Device for measuring at least one gas saturation, in particular, oxygen saturation, in the blood of a living body (1), comprising a sensor (23, 123) to be applied to the body, and light radiation means (27) for radiating light of different wavelengths from the sensor (23, 123) into the body (1) in at least one light radiation area (35), and further comprising light receiving means (29), the sensor (23, 123) having light receiving areas (45, 245) at different distances from the light radiation area (35, 135), in which areas (45, 245) it is possible for light scattered back in the body (1) to reach the light receiving means (29), the said means (29) being configured so as to determine the respective light intensity of the light reaching them in each light receiving area (45, 245), and electronic circuitry (91) connected with the light receiving means (29) being provided in order to determine the at least one gas saturation from the light intensities measured for the different wavelengths, **wherein** the electronic circuitry (91) is designed to select a processing area from the light receiving areas (45, 245) using a given criterion, and to process only light arriving at the light receiving means (29) in the selected processing area for determining the or at least one gas saturation.

2. Device according to claim 1, **wherein** the electronic circuitry (91) is designed to process a portion of the received light varying periodically with the pulse frequency of the blood flowing through the body (1), for determination of the gas saturation, and to select the processing area such that it contains that light receiving area (45, 245) in which the amplitude of the intensity of the said portion of light having one of the wavelengths provided, or the ratio between this amplitude and the maximum value or the mean value or the minimum value of the intensity of the total light received at the respective light receiving area (45, 245) and having the respective wavelength, assumes a maximum.

3. Device according to claim 1 or 2, **wherein** the light receiving means (29) are provided with several light receivers (41) for separate measurement of the intensities of the light entering them, and wherein at least one light receiver (41, 241) is assigned to each light receiving area (45, 245), and wherein the electronic circuitry (91) is designed to assign at least one and at most some of the light receivers (41, 241) to the processing area, the light receivers (41, 241) preferably being photo-semiconductors.

4. Device according to any of claims 1 to 3, **wherein** the sensor (23, 123) has at least three, preferably at least five, light receiving areas (45, 245), which are at different distances from the light radiation area (35, 135).

5. Device according to claim 4, **wherein** the light receiving areas (45, 245) form a row, which is in a straight line, at least if the side of the sensor (23, 123) to be applied to the body (1) is viewed from above, and wherein each light receiving area (45, 245) has at least one light admission field (44, 244) through which light is passed on to the light receiving means (29), and wherein the extension of each light admission field (44, 244) as measured in longitudinal direction of the row, is more than half, preferably at least two thirds, of the distance between the centre points of adjacent light admission fields (44, 244).

6. Device according to claim 5, **wherein** the extension of the light radiation area (35, 135) measured parallel to the longitudinal direction of the row of light receiving areas (45, 245) does not exceed 2 mm and is preferably not more than 1 mm, the extension of the light radiation area (35, 135) measured transversely to the said longitudinal direction preferably not exceeding 6 mm.

7. Device according to any of claims 1 to 6, **wherein** the light radiation means (27) are provided with light sources (31) located in the sensor (23) for the generation of light types of different wavelengths, the said light sources (31) preferably being semiconductor diodes.

8. Device according to any of claims 1 to 6, **wherein** the light radiation means (127) are provided with at least one light source (131) located outside of the sensor (123) for the generation of light of different wavelengths, which light source (131) is connected with the sensor (123) via light transmission means (132, 133, 134, 136) in such a way as to permit the types of light of different wavelengths to be radiated from the sensor (123) sequentially through one and the same surface of the light radiation area (135).

9. Device according to claim 7 or 8, **wherein** the light radiation means (27, 127) are provided with light transmission means (33, 133) located between the, or each, light source (31, 131) and the light radiation area (35).

10. Device according to any of claims 1 to 9, **wherein** the light receiving means (29) are provided with light transmission means (43) located between the light receiving areas (45, 245) and the light receivers (41, 241).

11. Device according to claim 9 or 10, **wherein** the centre points of the light radiation area (35, 135) and the light receiving areas (45, 245) are situated on a centre line (47), which is a straight line, at least in a view from above of the side of the sensor (23, 123) to be applied to the body (1), and wherein the light transmission means (33, 43) are provided with lightguides configured as plane disks (51) located in the sensor (23, 123), these lightguides being at a right angle to the centre line (47) in the said view from above.

12. Device according to any of claims 1 to 11, **wherein** the electronic circuitry (91) is designed to divide the length of the pulse period into several time intervals, and to accumulate separately for the different wavelengths the intensities of the light received by the light receiving means (27) during the different time intervals for a number of pulse periods.

13. Device according to any of claims 1 to 12, **wherein** the sensor is provided with at least one ultrasonic source (61) and ultrasound transmission means, in order to transmit ultrasonic waves generated by the ultrasonic source (61) to the living body (1), and wherein the ultrasound transmission means comprise an ultrasound transmission body (67), which presents an application surface (69) to be applied to the living body (1) and contains the light radiation area (35, 135) as well as all light receiving areas (45, 245), at least essentially.

14. Device according to claim 13, **wherein** the the ultrasound transmission body (67) is configured so as to transform into heat 30% to 70% of the energy received in the form of ultrasonic waves, and to transmit them to the living body (1), at least partly, by means of thermal conduction.

15. Device according to claim 13 or 14**, wherein** the ultrasonic source (61) comprises an electrode (64) used for generating ultrasonic waves, which is formed by an electric resistor serving as a temperature sensor at the same time.

16. Device according to any of claims 1 to 15, **wherein** a calibration-light distributor (81) is provided, which may be directed towards the light radiation area (35, 135) and towards the light receiving areas (45, 245) for calibration of the device, and is designed to distribute over the different light receiving areas (45, 245) the light arriving from the light radiation means (27, 127) in the light radiation area (35, 135), and to transmit such light to the light receiving means (29) in the said receiving areas (45, 245).

17. Device according to any of claims 1 to 16, **wherein** the light radiation means (27, 127) are designed for the generation of at least three types of monochromatic light, each of a different wavelength, and wherein one of these light types has a wavelength of 500 nm to 600 nm and is used to determine a quantity giving a measure for the volume of blood present in a section of the living body (1).

## Revendications

1. Dispositif pour la mesure d'au moins une saturation en gaz, notamment pour la saturation en oxygène du sang se trouvant dans un corps vivant (1) avec un détecteur (12, 123) prévu pour être appliqué sur le corps (1), avec des moyens (27) de rayonnement de lumière pour rayonner dans au moins une zone (35) de rayonnement de lumière, à partir du détecteur (23, 123) sur le corps (1), de la lumière comportant différentes longueurs d'ondes et avec des moyens (29) de réception de la lumière, tandis que le détecteur (23, 123) comporte des zones réceptrices de la lumière (45, 245) se trouvant à différentes distances de la zone (35, 135) de rayonnement de la lumière, et dans lesquelles la lumière récurrente dispersée sur le corps (1) peut parvenir aux moyens de réception de la lumière (27), ces derniers étant conçus pour déterminer, pour chaque zone réceptrice de la lumière (45, 245) l'intensité de la lumière qui leur parvient par elle, et des moyens de branchement (91) reliés aux moyens (29) de réception de la lumière étant prévus pour déterminer, à partir des intensités de lumière déterminées pour les différentes longueurs d'ondes, la saturation en gaz au moins unique,
dispositif caractérisé en ce que les moyens de branchement (91) sont conçus pour sélectionner sur la base d'un critère une zone d'utilisation à partir des zones réceptrices de lumière (45, 245), et pour n'utiliser que la lumière arrivant aux moyens de réception de la lumière (29) dans la zone d'utilisation sélectionnée pour la détermination d'au moins une saturation en gaz.

2. Dispositif selon la revendication 1, caractérisé en ce que les moyens de branchement (91) sont conçus pour utiliser, pour la détermination de la saturation en gaz, une partie de la lumière reçue, changeant périodiquement dans le temps avec la fréquence de pulsation du sang circulant à travers le corps (1), et pour déterminer la zone d'utilisation de façon que celle-ci comprenne la zone réceptrice de lumière (4b, 24b) pour laquelle l'amplitude de l'intensité de la, partie mentionnée de la lumière comportant une des longueurs d'onde, ou bien le rapport entre cette amplitude et la valeur maximale ou la valeur moyenne, ou la valeur minimale de l'intensité de la totalité de la lumière reçue dans la zone réceptrice de lumière (45, 245) en question et comportant la longueur d'onde mentionnée, est au maximum.

3. Dispositif selon la revendication 1 ou 2, caractérisé en ce que les moyens de réception de la lumière (29) comportent plusieurs récepteurs de lumière (41) pour la mesure séparée des intensités de la lumière arrivant sur eux, en ce que à chaque zone réceptrice de lumière (45, 245) est associé au moins un récepteur de lumière (41, 241) et en ce que les moyens de branchement (91) sont conçus pour associer à la zone d'utilisation au moins un et au plus, une partie des récepteurs de lumière (41, 241), les récepteurs de lumière (41, 241) étant de préférence constitués de photosemi-conducteurs.

4. Dispositif selon une des revendications 1 à 3, caractérisé en ce que le détecteur (23, 123) comporte au moins trois, aussi bien que de préférence au moins cinq zones réceptrices de lumière (45, 245) qui sont à des distances différentes de la zone (35, 135) de rayonnement de la lumière.

5. Dispositif selon la revendication 4, caractérisé en ce que les zones réceptrices de lumière (45, 24b) forment une rangée qui, au moins sur une vue de dessus, sur le côté du détecteur (23, 123) prévu pour être appliqué sur le corps (1), est rectiligne, en ce que chaque zone réceptrice de lumière (45, 245) a au moins un champ d'entrée de la lumière (44, 244), à travers lequel la lumière peut arriver dans les moyens de réception de la lumière (29), et en ce que l'extension mesurée en direction longitudinale de la rangée, de chaque champ d'entrée de la lumière (44, 244) est supérieure à la moitié et est de préférence au moins égale aux deux tiers de la distance des points médians de deux champs d'entrée de la lumière (44, 244) voisins l'un de l'autre.

6. Dispositif selon la revendication 5, caractérisé en ce que l'extension de la zone de rayonnement de la lumière (35, 135) mesurée parallèlement à la direction longitudinale de la rangée des zones réceptrices de la lumière (45, 245) est d'au plus 2 mm et de préférence d'au plus 1 mm, tandis que l'extension de la zone de rayonnement de la lumière (35, 135) est de préférence au plus de 6 mm.

7. Dispositif selon une des revendications 1 à 6, caractérisé en ce que les moyens de rayonnement de la lumière (27) comportent des sources de lumière (31) disposées dans le détecteur (23), et qui comportent des espèces de lumière pour produire les différentes longueurs d'ondes, tandis que les sources de lumière sont de préférence constituées de diodes semi-conductrices.

8. Dispositif selon une des revendications 1 à 6, caractérisé en ce que les moyens de rayonnement de la lumière (127), pour produire de la lumière de différentes longueurs d'ondes, comportent au moins une source de lumière (131) disposée en dehors du détecteur (123), qui par des moyens de transmission de la lumière (132, 133, 134, 136) est reliée au détecteur (123) de façon que les espèces de lumière comportant les différentes longueurs d'ondes puissent être rayonnées séquentiellement hors du détecteur (123) par une seule et même surface de la zone de rayonnement (135) de la lumière.

9. Dispositif selon la revendication 7 ou 8, caractérisé en ce que les moyens de rayonnement de la lumière (27, 127) comportent des moyens conducteurs de la lumière (33, 133) disposés entre chaque source de lumière (31, 131) et la zone de rayonnement de la lumière (35).

10. Dispositif selon une des revendications 1 à 9, caractérisé en ce que les moyens (29) de réception de la lumière comportent des moyens conducteurs de la lumière (43) disposés entre les zones réceptrices de la lumière (45, 245) et les récepteurs de lumière (41, 241).

11. Dispositif selon la revendication 9 ou 10, caractérisé en ce que les points médians de la zone (35, 135) de rayonnement de la lumière et des zones réceptrices de la lumière (45, 245) se situent sur une ligne médiane (47), qui au moins dans une vue de dessus sur le côté du détecteur (23, 123) prévu pour s'appliquer sur le corps (1) est rectiligne, et en ce que les moyens conducteurs de lumière (33, 43) comportent des conducteurs de lumière formés de plaques planes (51) qui, dans la vue de dessus précitée, sont à angle droit par rapport à la ligne médiane (47).

12. Dispositif selon une des revendications 1 à 11, caractérisé en ce que les moyens de branchement (91) sont prévus pour subdiviser la durée des périodes de pulsation en plusieurs intervalles de temps et pour cumuler séparément pour les différentes longueurs d'ondes, pendant plusieurs périodes de pulsation, les intensités de la lumière reçue par les moyens récepteurs de lumière (27) pendant les différents intervalles de temps.

13. Dispositif selon une des revendications 1 à 12, caractérisé en ce que le détecteur comporte au moins une source d'ultrasons (61) et des moyens de transmission d'ultrasons, pour transmettre les ondes ultrasonores produites par la source d'ultrasons (61) au cors vivant (1), en ce que les moyens de transmission d'ultrasons comportent un corps (67) de transmission d'ultrasons, qui forme une surface d'application (69) prévue pour être appliquée sur le corps vivant (1) et qui entoure, au moins pour l'essentiel, la zone de rayonnement de la lumière (35, 135) ainsi que toutes les zones réceptrices de la lumière (45, 245).

14. Dispositif selon la revendication 13, caractérisé en ce que le corps (67) de transmission d'ultrasons est prévu, pour convertir en chaleur 30 % à 70 % de l'énergie qui lui est amenée sous la forme d'ondes ultrasonores et pour la transmettre au moins partiellement par conduction thermique au corps vivant (1).

15. Dispositif selon la revendication 13 ou 14, caractérisé en ce que la source d'ultrasons (61) comporte une électrode (64) servant à produire les ondes ultrasonores et qui est constituée par une résistance électrique servant également de sonde de température.

16. Dispositif selon une des revendications 1 à 15, caractérisé par un répartiteur de lumière étalon (81) qui pour étalonner le dispositif peut être tourné vers la zone (35, 135) de rayonnement de la lumière ainsi que vers les zones réceptrices de la lumière (45, 245) et amener dans celles-ci aux moyens récepteurs de la lumière, la lumière qui lui est amenée à partir des moyens (27, 127) de rayonnement de la lumière dans la zone de rayonnement de la lumière (35, 135).

17. Dispositif selon une des revendications 1 à 16, caractérisé en ce que les moyens (27, 127) de rayonnement de la lumière sont prévus pour produire au moins trois espèces de lumière monochromatique, comportant chacune une autre longueur d'onde, et en ce que, une de ces espèces de lumière a une longueur d'onde de 500 nm à 600 nm et sert à déterminer une grandeur qui donne une mesure pour le volume du sang se trouvant dans une partie du corps vivant (1).
